# EUROPEAN PATENT APPLICATION

(11) **EP 1 980 254 A1**
(43) Date of publication of application: **15.10.2008**
(21) Application number: 07106191.5
(22) Date of filing: 13.04.2007
(51) Int. Cl.: A61K 31/568, A61K 38/17, A61P 25/28

(54) **Means and methods for counteracting protein aggregation**

(71) Applicant: Rijksuniversiteit te Groningen, 9712 CP Groningen (NL)
(72) Inventor: Kampinga, Harm Harmannus, 9791 KG, Ten Boer (NL); Maldonado, Maria Alexandra Rujano, 9713 TL, Groningen (NL); Hageman, Jurre, 9721 AE, Groningen (NL)
(74) Representative: van Loon, C.J.J.

(57) **Abstract**

The invention provides a use of a substance that is capable of enhancing the amount and/or activity of DnaJB8, or a functional part, derivative and/or analogue thereof, for counteracting protein aggregation.

## Description

The invention relates to the fields of biology and medicine.

Cellular protein aggregation is a process that acts in competition with normal protein folding processes. Proteins that are not correctly folded often aggregate and the presence of insoluble intracellular complexes result in a wide range of diseases, comprising, amongst other things, Alzheimer's disease, transmissible spongiform encephalopathies, Parkinson's disease, type 2 diabetes, transthyretin-mediated amyloid diseases such as familial amyloid cardiomyopathy and familial amyloidotic polyneuropathy, and amyotrophic lateral sclerosis.

A number of human genetic diseases are associated with an expansion of short tandem repeats in coding or non-coding gene regions. Examples comprise spinocerebellar ataxias type 10, which is associated with an expansion of a pentanucleotide repeat (ATTCT) in intron 9 of the SCA10 gene sequence to as many as 4500 copies; myotonic dystrophy type 1, an autosomal dominant multisystemic disorder characterized by the amplification of an unstable (CTG)n repeat in the 3'-untranslated region of a protein kinase gene; myotonic dystrophy type 2, characterized by expansion of a CCTG repeat in intron 1 of a zinc finger protein gene, and a group of neurodegenerative disorders or polyglutamine-mediated diseases, which are characterized by an expansion of a polyglutamine-encoding CAG repeat in a diversity of genes that are otherwise unrelated.

Protein misfolding and aggregation are also associated with cytotoxicity in polyglutamine diseases. Polyglutamine-mediated neurodegenerative disorders comprise to date a total of nine established neurodegenerative disorders, which are characterized by the genetic expansion of polyglutamine repeats in specified proteins. Clinical features and patterns of neuronal degeneration differ among the diseases but important pathogenic characteristics are common: all are progressive diseases characterized by neuronal dysfunction and neuronal loss progress over 10-30 years after onset, and are ultimately fatal. The underlying mechanism of polyglutamine-mediated neurotoxicity has not been fully elucidated. Expanded polyglutamine repeats are thought to result in conformational changes in the proteins that lead to misfolding, aggregation, inclusion body formation, and eventual neuronal cell death.

Polyglutamine-mediated neurodegenerative disorders comprise X-linked spinal and bulbar muscular atrophy (Kennedy disease), an X-linked recessive disorder caused by enlargement of a poly(Q) stretch in the androgen receptor protein; Huntington's disease, an autosomal dominant disorder caused by enlargement of a poly(Q) stretch in the huntingtin protein; dentatorubral-pallidoluysian atrophy (Haw River syndrome), an autosomal dominant disorder caused by enlargement of a poly(Q) stretch in the atrophin-1 protein; spinocerebellar ataxia type 1; an autosomal dominant disorder caused by enlargement of a poly(Q) stretch in the ataxin-1 protein; spinocerebellar ataxia type 2, an autosomal dominant disorder caused by enlargement of a poly(Q) stretch in the ataxin-2 protein;
spinocerebellar ataxia type 3 (Machado-Joseph disease), an autosomal dominant disorder caused by enlargement of a poly(Q) stretch in the ataxin-3 protein; spinocerebellar ataxia type 6; an autosomal dominant disorder caused by enlargement of a poly(Q) stretch in a voltage-dependent calcium channel subunit; spinocerebellar ataxia type 7, an autosomal dominant disorder caused by enlargement of a poly(Q) stretch in the ataxin-7 protein; and spinocerebellar ataxia type 17, an autosomal dominant disorder caused by enlargement of a poly(Q) stretch in a TATA binding protein.

The expansion of a polyglutamine-encoding CAG repeat results in the extension of a stretch of glutamines in the encoded proteins from between about 4-40 residues to between about 20-100 residues, whereby a pathological threshold depends on the neurodegenerative disorder. The age of onset of clinical manifestations is inversely correlated to the length of the polyglutamine expansion. Proteins with an enlarged stretch of polyglutamines tend to aggregate and neuronal intranuclear inclusions comprising such aggregates are found in distinct neuronal populations in diseased individuals, resulting in dysfunctionality and degeneration of the affected neurons. The identity of the affected neuronal populations is depending on the disorder. For example, in X-linked spinal and bulbar muscular atrophy lower motor neurons are primarily affected, resulting in progressive bulbar and proximal limb muscle weakness and atrophy (Wood et al. (2003) Neuropathology and Applied Neurobiology 29: 529-545).

Polyglutamine diseases are diseases of misfolding, in which the disease-related proteins are prone to aggregation. Insoluble intracellular protein aggregates are hallmarks of these disorders. This suggests that chaperones, the Ubiquitin Proteasome System (UPS) and other protein degradation systems could play a significant role in protection against the disease progression. Indeed, genes involved in RNA metabolism, protein synthesis, protein folding (such as chaperones), protein trafficking, regulators of the oxidative stress and components of the proteasome have been identified in screenings for modifiers of polyglutamine aggregation in C. elegans (Nollen et al., 2004) or neurodegeneration in Drosophila (Fernandez-Funez et al., 2000; Kazemi-Esfarjani and Benzer, 2000).

Molecular chaperones are a group of structurally diverse, evolutionary highly conserved proteins that interact with the non-native conformation of other proteins and mediate their folding or assembly, but are not components of the final functional structures (Frydman, 2001; Hartl and Hayer-Hartl, 2002). Chaperones are ubiquitously expressed and are found in all cellular compartments of the eukaryotic cell, which reflects their essential function under normal growth conditions. Despite their similar role in facilitating folding and assembly of proteins, some of their specific functions differ, and in many cases they act in tandem with eachother (Hartl and Hayer-Hartl, 2002). A large number of studies have been performed in order to determine the role of molecular chaperones in the pathogenesis of expanded polyglutamine proteins. Indeed, there are several reports that members of the Heat Shock Protein 70 (Hsp70) family and/or its cofactors can modulate polyglutamine aggregation and pathogenesis. Whereas in most cell models the Hsp70 machine reduces the extent of polyglutamine aggregation, it is still unclear what the crucial factors are.

Human molecular chaperones comprise Hsp 110, Hsp 70, and Hsp 40 heat shock protein family members, whereby each family is characterized by the presence of specific protein domains. The Hsp 110 family comprises 3 members, the Hsp 70 family comprises 11 members, whilst the Hsp 40 family comprises more than 40 members (see Table 1). Many studies have been performed in order to determine the role of molecular chaperones in protein aggregation events. However, which heat shock family members are effective in counteracting protein aggregation clearly differs between models. Furthermore, there are large differences in the efficacy of different kinds of molecular chaperones of counteracting protein aggregation and *in vitro* results are often not reproducible *in vivo*. For example, while components of the Hsp70 machine can reduce aggregation and pathogenesis in polyglutamine disease, the magnitude of effects, especially for Huntington's disease has been moderate. Also, no clear insight has been obtained on how the Hsp70 handles the polyglutamine-containing proteins. Furthermore, there are several indications that polyglutamine containing proteins are very poor substrates for the proteasome (Venkatraman et al., 2004; Holmberg et al., 2004).

It is therefore an object of the invention to provide improved means and methods for counteracting protein aggregation. It is a further object of the invention to provide means and methods for counteracting diseases associated with protein aggregation.

The present invention provides a use of a substance that is capable of enhancing the amount and/or activity of DnaJB8, or a functional part, derivative and/or analogue thereof for the preparation of a medicament for at least in part treating and/or preventing a disorder associated with protein aggregation. Said substance is preferably capable of enhancing the amount and/or activity of DnaJB8, or a functional part, derivative and/or analogue thereof, in a cell, preferably in a mammalian cell.

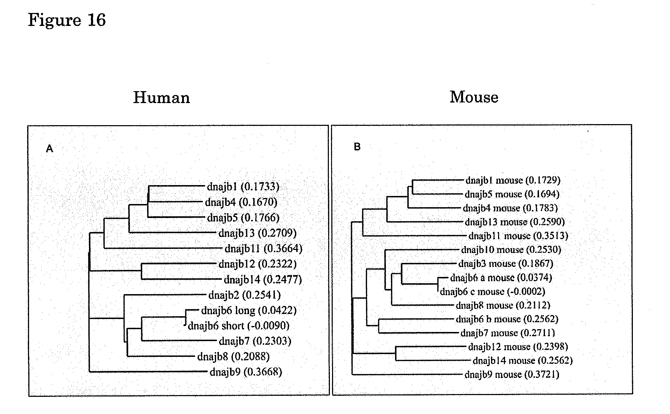

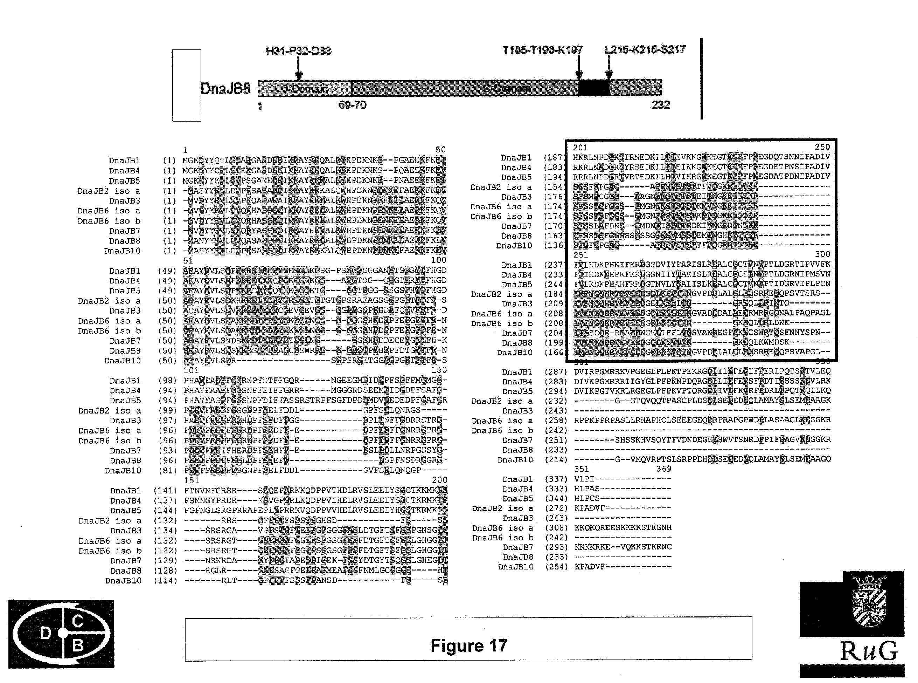

DnaJB8 is a member of the Hsp40 family (see also Figures 16 and 17). According to the present invention, DnaJB8 is particularly well capable of counteracting protein aggregation and toxicity mediated by protein aggregation. DnaJB8 was found to be the most effective chaperone as identified in the elaborate study of the present inventors. At equal expression levels, DnaJB8 was found to reduce the amounts of aggregates to a much higher extent than other Hsp family members. At equal amounts of Hsp-encoding nucleic acids, the presence of a nucleic acid encoding DnaJB8 results in more protein aggregate reduction as compared to nucleic acid encoding other Hsp proteins. Therefore, a substance that is capable of enhancing the amount and/or activity of DnaJB8, or a functional part, derivative and/or analogue thereof, is particularly suitable for counteracting protein aggregation.

The amount of DnaJB8 in a cell (also called the protein level of DnaJB8 in a cell) is defined herein as the amount of DnaJB8 protein and/or the amount of a functional part, derivative and/or analogue of DnaJB8 within a cell. Such amount is preferably expressed as µg DnaJB8 /cell.

A substance is defined herein as a natural or non-natural molecule or combination of molecules. A substance may comprise a compound, including but not limited to a small molecule compound such as, for example, a steroid. A substance may also comprise a peptide, a protein or a nucleic acid molecule, including but not limited to an expression vector, or any combination thereof.

Hsp 40 family members are homologous to the *Escherichia coli* DnaJ protein and contain a characteristic J-domain that mediates interaction with Hsp 70 and regulate ATPase activity by Hsp 70. However, according to the present invention, a DnaJB8 protein comprising a deletion in the J-domain that disrupts its potential to interact with Hsp70 is still able to counteract protein aggregation, showing that DnaJB8 acts independent of interaction with Hsp 70 family members. A major advantage of this finding is that it allows the development of protein aggregation inhibitors that work independently from HSP70, thereby bypassing a need for activating or upregulating Hsp70 which has been associated with enhanced tumorigenicity by preventing tumor cell death (see e.g. Rohde M, Daugaard M, Jensen MH, Helin K, Nylandsted J, Jaattela M. Members of the heat-shock protein 70 family promote cancer cell growth by distinct mechanisms. Genes Dev. 2005 Mar 1;19(5):570-82).

A disorder associated with protein aggregation is defined as a disorder that is characterized by an accumulation of aggregated protein, such as for instance Alzheimer's disease and transmissible spongiform encephalopathies, Parkinson's disease, type 2 diabetes, transthyretin-mediated amyloid diseases, amyotrophic lateral sclerosis (Lou Gehrig's Disease), and diseases characterized by expansions of a small nucleotide repeat, including but not limited to Friedreich's ataxia, Myotonic dystrophy types 1 and 2, and polyglutamine-mediated disorders.

In a preferred embodiment, a use according to the invention is provided wherein said disorder is associated with polyglutamine-mediated protein aggregation. Polyglutamine-mediated neurodegenerative disorders are described hereinbefore. In a particularly preferred embodiment, the invention provides a use according to the invention wherein said disorder is selected from Huntington's disease, dentatorubral-pallidoluysian atrophy, X-linked spinal and bulbar muscular atrophy, and spinocerebellar ataxias.

In another aspect, the invention provides a use of a substance that is capable of enhancing the amount and/or activity of DnaJB8, or a functional part, derivative and/or analogue thereof, for preventing aggregation of a protein.

The aggregation of proteins into insoluble intracellular complexes is a common problem in the production of proteins such as for instance therapeutic proteins. Therapeutic proteins are proteins that are engineered in the laboratory for pharmaceutical use and often comprise a recombinant protein. Therapeutic proteins are used to treat patients suffering from many conditions, including, but not limited to, cancer, Gaucher's disease, diabetes, anaemia, and haemophilia. Major therapeutic proteins comprise monoclonal antibodies, interferon, and erythropoietin. Other therapeutic proteins comprise insulin, blood clotting factors, vaccine antigens and soluble proteins including but not limited to growth hormones and interleukins. Aggregation of proteins during or after production involve, amongst other things, the risk of inability to manufacture said product, loss of biological activity such as loss of potency, and enhanced immunogenicity of said product. Enhanced immunogenicity is for instance caused by the high molecular weight of the aggregate and/or the fact that an aggregate displays repetitive epitopes.

Other areas that require the production of proteins, and especially purified proteins, and which will benefit from a reduction of aggregate formation include structural proteomics and the development and production of *in vitro* assays such as enzyme-linked immunoabsorbant assay and protein activity assays.

Proteins, including therapeutic proteins, can be produced in a cell-free system such as for instance a bacterial-derived *in vitro* expression system, a wheat germ cell-free protein translation system, a cell-free rabbit reticulocyte expression system, and an insect-based cell-free protein expression. Any system capable of producing a protein encoded by a nucleic acid sequence is called herein a nucleic acid expression system.

Proteins, including therapeutic proteins, can be expressed in either prokaryotic or eukaryotic cells, including cells from lower eukaryotes such as Saccharomyces cerevisiae, and Pichia pastoris. Proteins can be expressed in primary cells, such as oocytes, fibroblasts and kerotinocytes. Alternatively, they can be produced in cell lines including but not limited to mammalian cell lines such as Chinese hamster ovary cells, HEK 293 cells, COS-7 cells, HeLa cells, Vero cells, MCF7 cells, Madine Darbey canine kidney cells, and PER. C6 cells. Furthermore, proteins can be produced in whole organisms, including but not limited to a plant species such as Lemna gibba and Lemna minor, Nicotiana species, and Arabidopsis species, and animal species such as Mus musculus and Bos bovine.

In a preferred aspect, the invention provides the use of a substance that is capable of enhancing the amount and/or activity of DnaJB8, or a functional part, derivative and/or analogue thereof, in a cell for preventing aggregation of a protein in a mammalian cell.

The invention furthermore provides the use of a substance that is capable of enhancing the amount and/or activity of DnaJB8, or a functional part, derivative and/or analogue thereof, for preventing aggregation of a protein whereby preventing aggregation results in enhanced recovery of said protein. Optimizing the levels of soluble protein is an attractive strategy to increase pure and active protein yield compared to recovering highly expressed protein in aggregated form. Recovery of aggregated proteins is usually poor and often affects the integrity and activity of the recovered protein. In addition, purification of over-expressed soluble proteins is faster and cheaper than obtaining it from aggregated forms.

In a preferred embodiment, a substance according to the invention comprises DnaJB8 protein, or a functional part, derivative and/or analogue thereof. According to the invention, the presence of DnaJB8, or a functional part, derivative and/or analogue thereof, efficiently counteracts protein aggregation *in vitro* and *in vivo*, prevents cellular toxicity in cell systems, and allows normal development of Xenopus embryos expressing an expanded polyglutamine protein into tadpoles.

In one embodiment the amount of DnaJB8 is enhanced by enhancing expression of endogenous DnaJB8 of a cell. Alternatively, or additionally, DnaJB8 or a functional part, derivative and/or analogue thereof is administered to a cell and/or an individual. Alternatively, or additionally, a nucleic acid sequence encoding DnaJB8 or a functional part, derivative and/or analogue thereof is administered to a cell and/or an individual.

DnaJB8 or a functional part, derivative and/or analogue thereof, and/or a nucleic acid sequence encoding therefore, is administered to an individual by any method known in the art, for instance, but not limited to, oral administration and/or injection, for instance infusion. Emerging methods to deliver pharmaceutical substances comprise controlled delivery technologies, including local delivery technologies, needle-free systems, and pulmonary inhaler systems, which can also be used for administering DnaJB8 protein, or a functional part, derivative and/or analogue thereof, or a nucleic acid encoding DnaJB8 or a functional part, derivative and/or analogue thereof. The DnaJB8 protein, or a functional part, derivative and/or analogue thereof, or a nucleic acid encoding therefore, is preferably administered together with a pharmaceutically acceptable carrier, diluent or excipient.

DnaJB8 is directly administered to a cell using any known method, such as for instance injection and/or electroporation. Nucleic acid encoding DnaJB8 or a functional part, derivative and/or analogue thereof is for instance administered to a cell using plasmids, for instance in virosomes, and/or using viral vectors such as for instance an adenoviral, lentiviral or retroviral vector. In one embodiment a nucleic acid encoding DnaJB8 or a functional part, derivative and/or analogue thereof is administered to a cell using lipoplexes. A preferred method comprises expression of nucleic acid molecules encoding a plurality of light chain molecules or functional parts thereof, which method is discussed hereinafter.

A preferred substance for use according to the invention comprises a small molecule compound. Small molecules have good absorption and permeation in biological systems and are consequently more often successful drug candidates than compounds with a molecular weight above 500 Dalton (Lipinski et al. (1997) Adv Drug Deliv Rev 23: 3-25). Small molecule compounds have interesting agonistic or antagonistic functions that interfere with a biological process of interest. In one preferred embodiment a small molecule compound is used which is capable of enhancing the activity of endogenous DnaJB8.

Methods to enhance an expression level of an endogenous protein are known in the art. Well known methods comprise the administration of a naturally occurring factor, including but not limited to a lipid, a steroid, or a vitamin. A method for enhancing endogenous expression level of DnaJB8 for instance comprises stimulation of a transcriptional activator or inhibition of a transcriptional repressor.

In a preferred embodiment, expression of DnaJB8 is enhanced by administration of a steroid, such as for instance, but not limited to, a sex steroid, such as estrogen, progesterone and androgen, and/or a corticosteroid such as for instance glucocorticoid and/or mineralocorticoid.

In a more preferred embodiment, said steroid is testosterone or a functional part, derivative and/or analogue thereof. Said testosterone may comprise unmodified testosterone or modified testosterone such as for instance 17-alpha alkyltestosterones, for instance methyltestosterone, fluoxymesterone, testosterone enanthate, and/or testosterone cypionate (Depo- Testosterone).

Further provided is therefore a use of testosterone or a functional part, derivative and/or analogue thereof for the preparation of a medicament for treating a disorder associated with protein aggregation. Said disorder is preferably associated with polyglutamine-mediated protein aggregation. In a particularly preferred embodiment the use of testosterone for the preparation of a medicament for treating a disorder selected from Huntington's disease, dentatorubral-pallidoluysian atrophy, X-linked spinal and bulbar muscular atrophy, and spinocerebellar ataxias is provided.

In an alternative embodiment, a method to enhance the endogenous expression level of DnaJB8 comprises the use of an artificial factor such as for instance a chimeric modified zinc finger protein that is for instance coupled to a transcriptional activator domain and is for instance designed to bind to a region in the promoter of a gene encoding DnaJB8 protein (Segal et al. (1999) PNAS 96: 2758-2763). In another embodiment the expression of a gene encoding DnaJB8 protein is enhanced by lowering the effective amount and/or activity of a repressor of said gene, by for example, expression of siRNA, double stranded RNA, antisense RNA, or a ribozyme such as a hammerhead ribozyme.

As used herein, the term analogue refers to an isoform of a DnaJB8 protein, which is for instance isolated from vertebrates such as from mouse, bovine, or chicken. An isoform can also be provided by, for example, conservative amino acid substitution.
The term derivative refers to a modified form of a DnaJB8 protein, including but not limited to a glycosylated form and/or a pegylated form, which may improve the pharmacological properties of a protein drug and may also expand its half life. A functional part of DnaJB8 is defined herein as a part which has the same properties in kind, not necessarily in amount. A functional part of DnaJB8 has the same capability of counteracting protein aggregation as DnaJB8, albeit not necessarily to the same extent.

Said DnaJB8 protein, or a functional part, derivative and/or analogue, can be produced in a cell-free system, or a prokaryotic or eukaryotic cell, including but not limited to a mammalian cell as described hereinabove.

A preferred substance according to the invention comprises the C-domain of the DnaJB8 protein, which constitutes the C-terminal part of the protein. The presence of this part was shown to be sufficient for preventing aggregation of a extended poly(Q)-containing protein.

Said C-terminal part of DNAJB8 preferably comprises the amino acid sequence GAFSAGFGEFPAFMEAFSSFNMLGCSGGSHTTFSSTSFGGSSSGSSGFKSVM SSTEMINGHKVTTKRIVENGQERVEVEEDGQLKSVTVNGKEQLKWMDSK.

Further provided is therefore ea use according to the invention, wherein said substances comprises the amino acid sequence
GAFSAGFGEFPAFMEAFSSFNMLGCSGGSHTTFSSTSFGGSSSGSSGFKSVM SSTEMINGHKVTTKRIVENGQERVEVEEDGQLKSVTVNGKEQLKWMDSK.
In another preferred embodiment, said substance comprises the amino acid sequence
VMSSTEMINGHKVTTKRIVENGQERVEVEEDGQLKSVTVNGKEQLKWMDSK.

In a further preferred embodiment a substance of the invention comprises a nucleic acid encoding a DnaJB8 protein, or a functional part, derivative and/or analogue thereof. Said nucleic acid is particularly suitable for expression of an exogenous DnaJB8 protein, or a functional part, derivative and/or analogue thereof. Said nucleic acid preferably comprises a promoter and/or termination sequence allowing expression of said DnaJB8 protein, or a functional part, derivative and/or analogue thereof.
Examples of preferred nucleic acid molecules include, but are not limited to, an RNA molecule, a DNA molecule such as, for example, a plasmid, or a virus such as, but not limited to, a retrovirus, an adenovirus, an adeno-associated virus, a lentivirus, and a herpes simplex virus. Nucleic acid analogues such as for instance peptide nucleic acid are also encompassed by the term "nucleic acid".

A particularly preferred nucleic acid expression unit comprises a plasmid, which preferably comprises a nucleic acid molecule comprising a promoter, a nucleic acid sequence encoding DnaJB8 or a functional part, derivative and/or analogue thereof and, optionally, a marker gene.
Said promoter region preferably comprises regulatory sequences that control the expression from said expression unit. Suitable promoter sequences are known in the art, including, but not limiting to, promoter sequences from a virus such as cytomegalovirus (CMV), or a promoter region from a housekeeping gene such as beta-actin. Alternatively, neuron-specific promoter sequences can be used to drive expression of DnaJB8 protein, or a functional part, derivative and/or analogue thereof in neuronal cells. Examples of neuron-specific promoter sequences comprise promoter sequences of Microtubule-Associated Protein 1B Gene, neurofilament gene, gonadotropin-releasing hormone gene, and synapsin I gene.

Said termination sequences for instance comprise termination sequences, including a poly(A) signal, from the DnaJB8 gene. Alternatively, said termination sequences may be derived from other genes, including but not limited to growth hormone gene and/or the gastrin gene.

Now that the invention provides the insight that DnaJB8 is particularly well capable of counteracting protein aggregation, it has become possible to screen candidate substances for their (indirect) capability of counteracting protein aggregation. A substance that is capable of enhancing the amount and/or activity of DnaJB8, or a functional part, derivative and/or analogue thereof, is (indirectly) capable of counteracting protein aggregation. Further provide is therefore a method for determining whether a candidate substance is capable of counteracting protein aggregation, comprising determining whether said candidate compound is capable of enhancing the amount and/or activity of DnaJB8, or a functional part, derivative and/or analogue thereof, preferably in a cell. Further provided is a method for determining whether a candidate substance is capable of counteracting protein aggregation, comprising determining whether said candidate substance is capable of enhancing expression and/or activity of DnaJB8, or a functional part, derivative and/or analogue thereof.

In another aspect, the invention provides a method for determining whether a substance is capable of enhancing the level of expression of DnaJB8, or a functional part, derivative and/or analogue thereof, in a mammalian cell, the method comprising providing a plurality of substances; providing at least one nucleic acid expression system comprising at least one nucleic acid molecule comprising a promoter region of DnaJB8 that is operationally linked to a reporter gene; contacting said at least one nucleic acid expression system with said plurality of substances; measuring expression of said reporter gene; and determining whether expression of said reporter gene is enhanced in the presence of a substance, as compared to the expression of said reporter gene in the absence of said substance, whereby an enhanced expression of said reporter gene in the presence of said substance demonstrates that said substance is capable of enhancing the level of expression of DnaJB8, or a functional part, derivative and/or analogue thereof.

A method according to the invention preferably further comprises selecting a candidate substance which is capable of enhancing expression and/or activity of DnaJB8, or a functional part, derivative and/or analogue thereof.

Said plurality of substances comprises natural and/or non-natural substances, including but not limited to libraries of compounds that are commercially available such as peptide libraries (e. g. MatchMaker^{™}; Clontech), lipid libraries (BioMol), synthetic compound libraries (e. g. LOPAC^{™}, Sigma Aldrich) or natural compound libraries.

Preferably the compounds are of low molecular weight, i. e. with a molecular weight of 500 Dalton or less. Such compounds often have good absorption and permeation in biological systems and are consequently more often successful drug candidates than compounds with a molecular weight above 500 Dalton (Lipinski et al. (1997)).

A reporter gene preferably comprises a selectable marker of which the level of expression or activity can be quantified. Reporter genes are known in the art and include LacZ encoding β-galactosidase, jellyfish green fluorescent protein gene, chloramphenicol acetyltransferase gene, alkaline phosphatase, and luciferase. Variants of these proteins, such as yellow fluorescent protein gene and variants with a shortened or prolonged half-life, can furthermore be used as reporter gene.

One preferred embodiment comprises determining whether a candidate compound is capable of enhancing the amount and/or activity of DnaJB8, or a functional part, derivative and/or analogue thereof, in a mammalian cell. A preferred mammalian cell is a cell in which the promoter region of DnaJB8 is moderately active such that enhancement of the expression of the reporter gene can be monitored. Furthermore, said nucleic acid expression unit comprising a promoter region of DnaJB8 that is operationally linked to a reporter gene is preferably transferable in said mammalian cell. Methods for transferring a nucleic acid expression unit are known in the art and include transfection, either transient or stable transfection, electroporation, and transduction comprising the use of a virus such as a retrovirus or an adenovirus. Suitable transfection agents include lipofectamine, polyethylenimine (PEI), and Fugene.

Now that the present invention provides the insight that type II Hsp40 proteins such as DnaJB8 are capable of counteracting protein aggregation without interaction with Hsp70 proteins, the need for activating or upregulating Hsp70 has been bypassed. This is favourable because Hsp70 has been associated with enhanced tumorigenicity. Hence, protein aggregation is preferably counteracted without activating or upregulating Hsp70. This has become possible with the insight provided by the present invention. According to the present invention, protein aggregation is preferably counteracted using a type II Hsp40 protein which is capable of acting independent of Hsp70. Preferably protein aggregation is counteracted by DnaJB8 or a functional part, derivative and/or analogue thereof. The invention furthermore provides means and methods for screening candidate compounds for their capability of enhancing a type II Hsp40 protein's capability of counteracting protein aggregation independent of Hsp70. One embodiment provides a method for determining whether a candidate substance is capable of enhancing a type II Hsp40 protein's capability of counteracting protein aggregation without interaction with or upregulation of a member of the HSP-70 family, the method comprising determining whether the anti protein aggregation activity of said type II Hsp40 protein, or of a functional part, derivative and/or analogue thereof, is enhanced in the presence of said candidate compound, as compared to the anti protein aggregation activity of said type II Hsp40 protein or functional part, derivative and/or analogue in the absence of said candidate compound, in a manner independent of interaction with or upregulation of members of the HSP-70 protein family. In one embodiment a cell-free environment essentially free of Hsp70 is used. Said type II Hsp40 protein preferably comprises DnaJB8 or DnaJB6. Most preferably, said type II Hsp40 protein comprises DnaJB8.
When a candidate substance appears to be capable of enhancing a type II Hsp40 protein's capability of counteracting protein aggregation without interaction with or upregulation of a member of the HSP-70 family, it is preferably selected. Said candidate substance is preferably further used and/or optimised for inhibiting protein aggregation via its influence on said type II Hsp40 protein or a functional part, derivative and/or analogue thereof.

In yet a further embodiment, the invention provides a method for determining whether a candidate substance is, without requiring interaction with or upregulation of members of the HSP-70 protein family, capable of enhancing the anti- polyglutamine-mediated protein aggregation activity of a type II Hsp40 protein, or of a functional part, derivative and/or analogue thereof, the method comprising:
a. providing a plurality of substances;
b. providing a multitude of receptacles, each comprising a mixture comprising a proteinaceous molecule comprising a stretch of more than 20 glutamines, and a type II Hsp40 protein;
c. adding said plurality of substances to said multitude of receptacles;
d. determining whether a substance is capable of decreasing the aggregation of said proteinaceous molecule in a manner independent of interaction with or upregulation of members of the HSP-70 protein family.
In one embodiment a cell-free environment essentially free of Hsp70 is used. Said type II Hsp40 protein preferably comprises DnaJB8 or DnaJB6. Most preferably, said type II Hsp40 protein comprises DnaJB8.
When a candidate substance appears to be capable of enhancing the anti-polyglutamine-mediated protein aggregation activity of a type II Hsp40 protein, or of a functional part, derivative and/or analogue thereof, it is preferably selected. Said candidate substance is preferably further used and/or optimised for inhibiting polyglutamine-mediated protein aggregation via its influence on said type II Hsp40 protein or a functional part, derivative and/or analogue thereof.

A receptacle refers to any container that is capable of holding a mixture, and for instance comprises an Eppendorf cup, or a tube. A multitude of receptacles comprises, for example, a multiwell plate such as a 96 well plate or a 384 well plate.

A proteinaceous molecule comprising a stretch of more than 20 glutamines refers to a natural protein that contains a stretch of more than 20 glutamines, such as, for example, Huntingtin protein or a relevant part thereof that comprises said stretch of glutamines, or any other kind of proteinaceous molecule, such as for instance a chimeric protein in which a fragment of exon-1 of Huntingtin protein is fused to a reporter protein, such as for example enhanced yellow fluorescent protein.

The invention furthermore provides a method for treating an individual suffering from, or at risk of suffering from, a disorder associated with protein aggregation, the method comprising administering to said individual a pharmaceutically effective amount of a substance that is capable of enhancing the amount and/or activity of DnaJB8, or a functional equivalent thereof, in said individual.

The invention is further explained in the following examples. These examples do not limit the scope of the invention, but merely serve to clarify the invention.

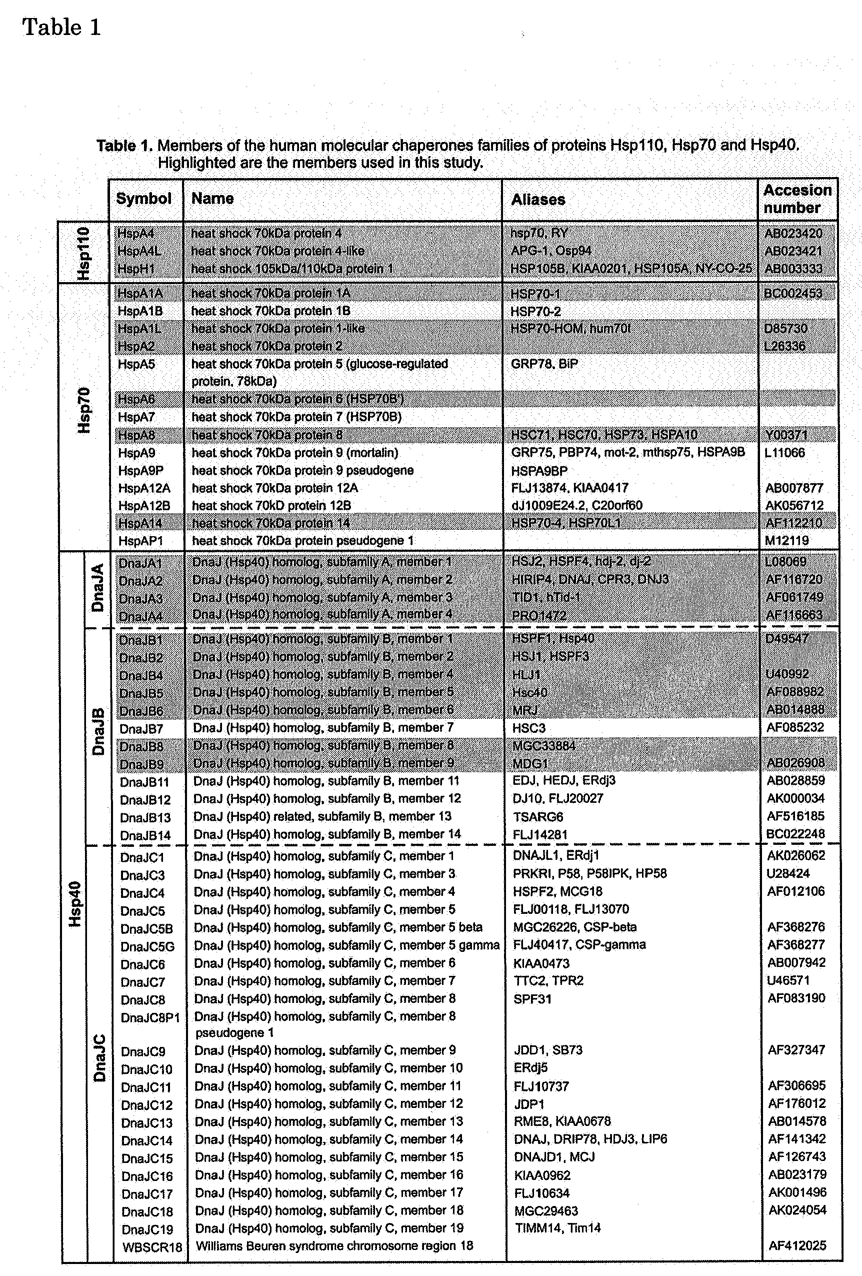

### Examples

### Example 1

### Materials and methods of Examples 1-7

**Cell culture and transient infections.** Flp-In T-Rex HEK293 cells (Human embryonic kidney 293) stably expressing the tetracycline (tet) repressor were obtained from Invitrogen. Cells were cultured in DMEM (Gibco) supplemented with 10% foetal bovine serum (Sigma) and 100 units/ml penicillin and 100µg/ml streptomycin (Invitrogen). 5 µg/ml Blasticidine (Sigma) and 100 µg/ml of Zeocin (Invitrogen) were added to the cultures. Cultures were maintained at 37°C and 5% CO2 in a humidified incubator. For transient transfections, cells were grown to 50-60% confluence in 35 mm-diameter dishes coated with 0.001% of poly-L-lysine (Sigma) and on coated coverslips for confocal microscopy analyses. Cells were transfected with a total of 1 µg of DNA using Lipofectamine (Gibco) according to the manufacturer instructions.

**Plasmids.** The construction ofpHDQ119-EYFP driving the expression of a fragment of exon-1 of huntingtin fused to the enhanced yellow fluorescent protein was previously described (Rujano et al., 2006). The construction of the tetracycline inducible HSP overexpression plasmids is described by Hageman et al. (manuscript in preparation). Briefly, first the V5 sequence containing a Kozak initiation codon and lacking a stop codon was inserted in pcDNA5/FRT/TO by oligo cloning. Subsequently, the coding sequence of each gene was amplified and the PCR products where purified, cleaved with the respective enzymes and ligated in frame in the pcDNAS/FRT/TO-V5 vector cleaved with the same set of enzymes. Presence of the correct insert was verified using DNA sequencing. Expression of the proteins at the expected molecular mass was verified by Western blot analysis against the V5-tag.

**Semi-quantitative RT-PCR.** Total RNA was extracted from the following cell lines: HEK-293 (CRL-1573), HeLA (CCL-2), A549 (CCL-185), HepG2 (HB-8065), SH-SY5Y (CRL-2266) and VH-25 (primary human foreskin fibroblasts). qPCR reference RNA and various post mortem tissue RNA samples were purchased from Stratagene. First strand cDNA was prepared as previously described (Hageman et al., 2005). During PCR, samples were taken after 20, 25, 30 and/or 35 cycles. PCR conditions were: 2 min at 94°C, followed by 30 sec at 94°C, 30 sec at 55°C and 30 sec at 72°C. For the amplification of DnaJB6 short and long isoforms, DnaJB8 and GAPDH as control housekeeping gene, the following primers were used: DnaJB6-short-for CTCATCGGAGCCTCTATTTG, DnaJB6-short-rev CTACGTGGCTCACATTTCAG, DnaJB6-long-for TGAAAGAAGGTGGCAAGAGG, DnaJB6-long-rev
GATCCGTGATCGCATAATCC, DnaJB8-for CCCGGAGGACATCAAGAAAG, DnaJB8-rev AGGTGTAGCCGGTGTCGAAG. GAPDH-for GTCCATGCCATCACTGCCAC, GAPDH-rev CATACCAGGAAATGAGCTT, PCR fragments were cloned and sequenced to verify the amplification of the correct products.

**Cell extracts and sample preparation.** 24 hours after transfection cells were recovered by trypsinization, pelleted and resuspended in 1 ml of PBS. The cell suspension was centrifuged at 6000 rpm for 5 min at RT and the pellet was resuspended in 75 µl of RIPA buffer containing 2% SDS supplemented with protease inhibitors and sonicated. Protein content was determined with the DC protein essay (Bio-RAD). Western blot samples were prepared at a final concentration of 1µg/µl in SDS-PAGE loading buffer and heated for 5 min at 100 °C. Filter trap samples were prepared at a final concentration of 100 ng/µl, 20 ng/µl and 4 ng/µl in FTA buffer (10 mM Tris-Cl pH 8.0, 150 mM NaCl and 50 mM dithiothreitol) + 2% SDS and heated for 5 min at 100 °C. Samples were used immediately or kept frozen at -20°C.

**Western blot analysis.** Equal amounts of protein were loaded on 10% or 12,5% SDS-PAGE gels. Proteins were transferred onto nitrocellulose membranes and probed with monoclonal anti-GFP antibody GL-8 (Clontech) at a 1:5000 dilution and monoclonal anti-V5 antibody (Invitrogen) at a 1:5000 dilution. GAPDH was used a loading control and was detected with a monoclonal antibody (RDI Research Diagnostics) at 1:1000 dilution. Blots were incubated with HRP-conjugated anti-mouse secondary antibody (Amersham) at 1:5000 dilution, and visualization was made using enhanced chemiluminescence and Hyperfilm (ECL, Amersham).

**Filter trap assay.** Filter trap assay was performed based on the protocol described by Carra et al. (2005). Briefly, 10, 2 and 0.4 µg of protein extracts were applied onto a 0,2 µm pore Cellulose Acetate membrane prewashed with FTA + 0.1% SDS. Mild suction was applied and the membrane was washed 3 times with the same buffer. Aggregated proteins trapped in the membrane were probed with monoclonal anti-GFP antibody GL-8 (Clontech) at a 1:5000 dilution and monoclonal anti-V5 antibody (Invitrogen) at a 1:5000 dilution followed by HRP-conjugated anti-mouse secondary antibody (Amersham) at 1:5000 dilution, and visualization was performed using enhanced chemiluminescence and Hyperfilm (ECL, Amersham). For quantitative analysis, relative intensity of the bands was measured using GelPro Analyzer 4.5 gel analyzer software.

**Immunolabeling and confocal microscopy.** 16-24 hours after transfection indirect immunofluorescence of the V5 tag was performed to detect the chaperones. Cells were fixed with 3.7% formaldehyde for 15 minutes, washed three times with Phosphate-Buffered Saline (PBS), permeabilized with 0.2% Triton-X100 and blocked during 30 minutes with 0.5% BSA and 0.1% glycine in PBS. Incubation with mouse anti-V5 monoclonal antibody (Invitrogen) 1/100 dilution was performed overnight at 4°C followed by a 1 hour incubation with CY5-conjugated anti-mouse secondary antibody (Jackson) at 1:200 dilution. To visualize nuclei, cells were stained 10 minutes with 0.2µg/ml 4',6-diamidino-2-phenylindole (DAPI). Coverslips were mounted in antifadent solution (10% Mowiol 40-88 (Sigma), 2.5% 1,4-Diazabicyclo[2.2.2]octane (DABCO, Sigma), 25% glycerol in 0.1 M Tris-HCl pH 8.5). Images of EYFP, CY3, and DAPI fluorescence were obtained using the Leica confocal laser scanning microscope (Leica TCS SP2, DM RXE) with a 63X/1.32 oil lens. The captured images were processed using Leica Confocal Software and Adobe Photoshop.

**Fluorometry.** 1 x 10⁵ GFP expressing cells where plated on a 96 well optical plate (Costar, NY, USA) in PBS. Relative GFP fluorescence was measured in a microplate fluorescence reader (Bio-Tek instruments, Inc, Vermont, USA) at an excitation wavelength/bandwidth of 485/20 nm and an emission wavelength/bandwidth of 516/20 nm.

**Flow cytometry.** For cell viability assays, propidium iodide (PI, 1 µg/ml, Sigma-Aldrich) was added to the cells 48 and 96 hours after transfection. Cells were collected and analyzed by flow cytometry on a FACS Calibur flow cytometer (Becton Dickinson, Erembodegem Germany) counting 10,000 cells in each group. Percentage of GFP positive cells that incorporate the dye (GFP + / PI +) represents percentage of dead cells.

### Results

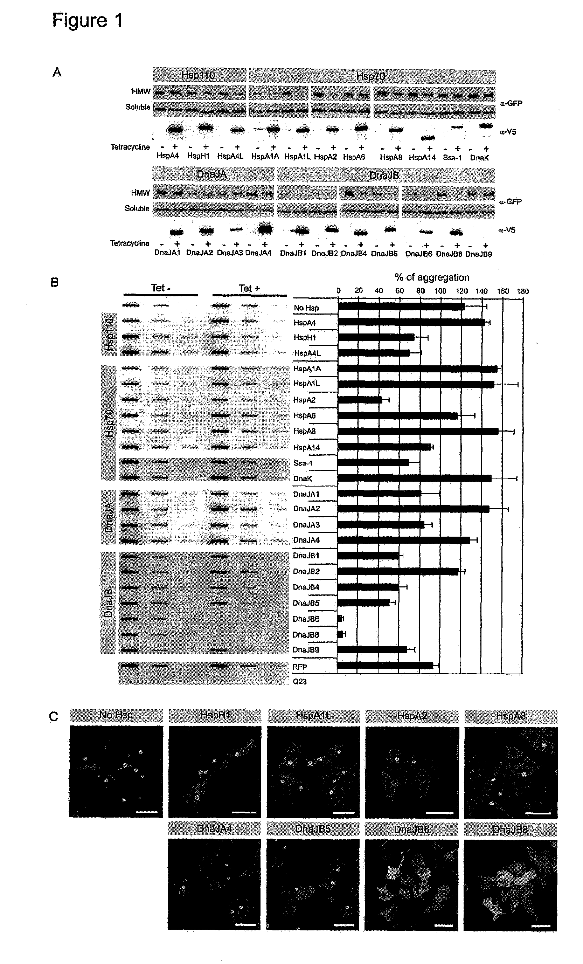

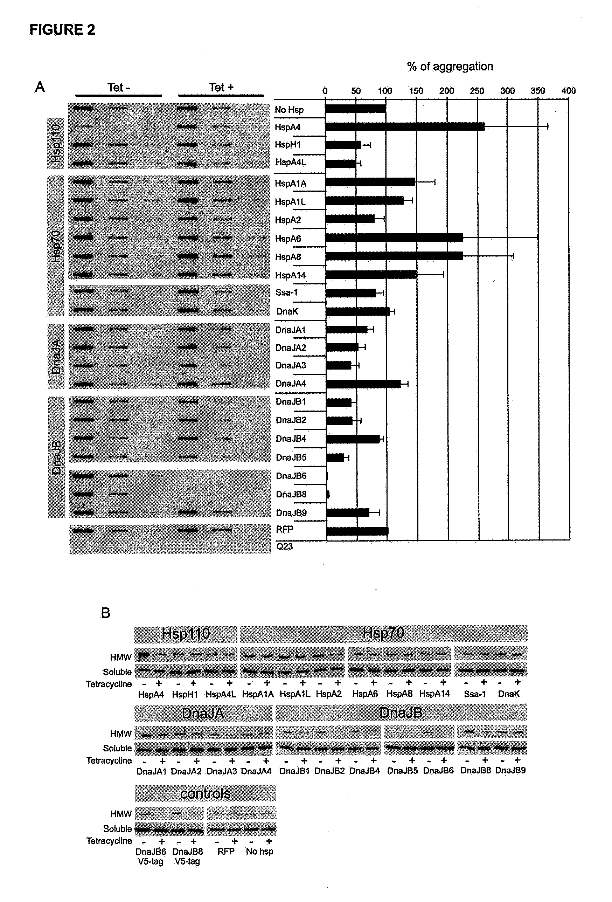

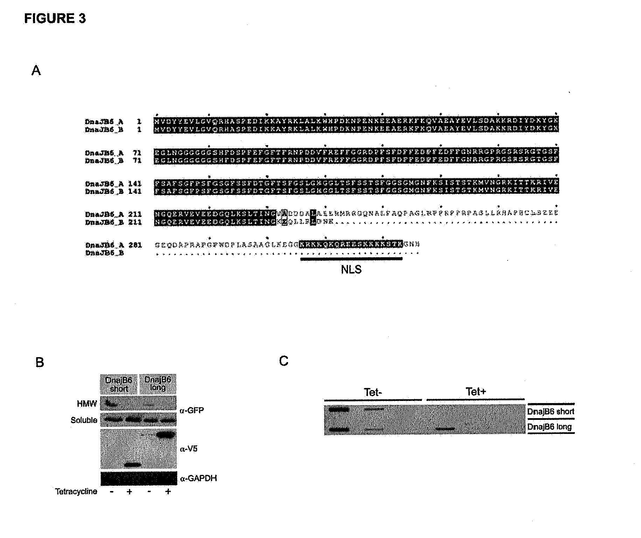

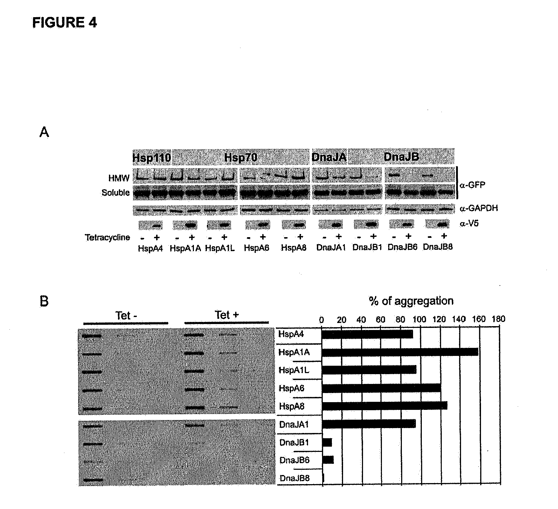

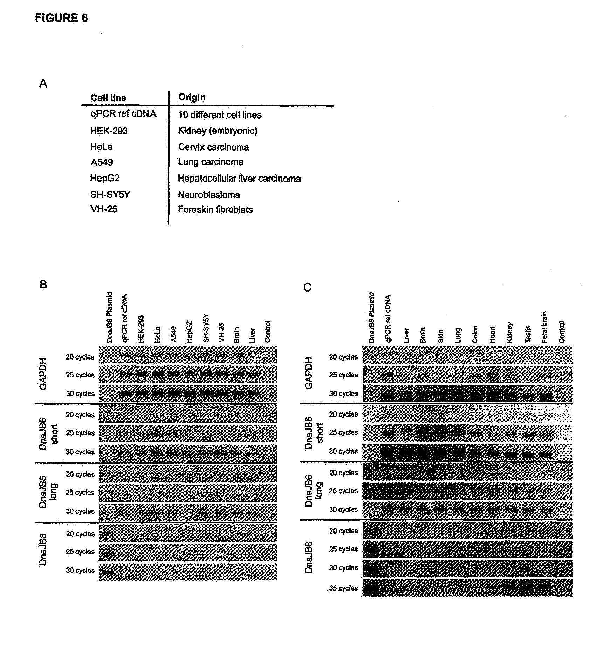

There are several members of each family of molecular chaperones in humans: the Hsp70 family of proteins comprises an abundant and highly conserved group of 11 members; the Hsp40 protein family has over 40 members and the Hsp110 family has 3 members (Table 1). To search for members of the molecular chaperones family of proteins that modulate the aggregation process of polyglutamine proteins, we cotransfected HEK293 cells with pHDQ119-EYFP (fluorescently tagged fragment of the exon-1 of the huntingtin gene containing 119 glutamines: HDQ119) and tetracycline inducible, V5-tagged members of Hsp110, Hsp70 and Hsp40 families (Table I). Expression of the chaperones was induced 2 hours after cotransfection with pHDQ119-EYFP by adding tetracycline containing fresh medium to the cells (Fig. 1A). Polyglutamine aggregation and expression of the transfected proteins was assessed by immunofluorescence and on cell extracts prepared 24 hours after transfection using filter trap assays and western blotting. Within the Hsp110 family, HspH1 and HspA4L showed a slight decrease in the aggregation of HDQ119, whilst HspA4 enhanced polyglutamine aggregation (Fig. 1A-C). Amongst the members in the Hsp70 family, only HspA2 significantly decreased the number of cells with aggregates (Fig. 1C). HspA2 also reduced the amount of aggregated proteins trapped in the slot blot (Fig. 1B) and high molecular weight aggregates as detected in the stacking gel after SDS-PAGE (Fig. 1A). Likewise, the yeast SSA-1 slightly diminished the aggregates trapped in the slot blot. Surprisingly, most other members of the Hsp70 family members had either no effect (HspA6 or HspA14) or even enhanced polyglutamine aggregation (HspA1A, HspA1L, HspA8, and E. coli DnaK). The latter mammalian Hsp's were efficient in enhancing refolding of heat denatured proteins, as determined by a cellular firefly luciferase refolding assay (data not shown). So, it can be concluded that different members of the Hsp70 family or its larger Hsp110 subfamily can have different effects on aggregation of HDQ 119, but none of the effects exceeded a factor of 1.5.
Next, we tested a subgroup of the large Hsp40 family proteins of either Type I (DnaJA: closest homology to the E.coli DnaJ) or Type II (DnaJB) (Cheetham and Caplan, 1998). Type I members were suggested to have substrate binding capacities of their own via their putative peptide binding domain that is lacking in the type II family members. Yet, none of the DnaJA proteins tested showed any significant effect on aggregation (Fig. 1A-C). In contrast, except for DnaJB2, members of the DnaJB protein family tested were effective in suppressing aggregation. DnaJB1, DnaJB4 and DnaJB5 showed a significant decrease in the number of cells with aggregates (Fig. 1C) and reduced the fraction of aggregates trapped in the slot blot (Fig. 1B) and present in the stacking gel (Fig. 1A). The largest anti-aggregation effect, however, was seen after overexpression of DnaJB6 and DnaJB8: both proteins caused an almost complete inhibition of aggregation of HDQ119 as judged by both fluorescence microscopy (Fig. 1C) and biochemical aggregation assays (Fig. 1A-B). These results amongst family members of the Hsp70 machine demonstrate that distinct members differentially affect the aggregation state of polyglutamine proteins. Furthermore, DnaJB6 and DnaJB8 are the most potent inhibitors.
In order to compare the efficacy of the different family members, we used V5-tagged constructs to be able to examine the expression levels of the transfected chaperones. Except for DnaJB9 which was poorly expressed, the expression levels were comparable for most family members (Fig. 1A). DnaJB6 and DnaJB8 were not expressed at higher levels than the other members to an extent that it could explain their exclusively large effect on aggregation of HDQ 119. However, it is possible that the V5-tags may have affected the properties of the chaperones (both negatively and positively). Therefore, the assay was repeated with untagged proteins with the assumption that the expression levels obtained would be the same as with the V5-tagged proteins. For most proteins tested, the results obtained with the untagged versions were qualitatively comparable and mostly more pronounced that those obtained with the V5-tag chaperones (Fig. 2A-B). An exception formed the class A members of the Hsp40 proteins of which DnaJA1, DnaJA2, and DnaJA3 now also showed some suppression of polyglutamine aggregation, suggesting that the V5-tag partially had affected their activity. Most importantly, however, also for the untagged chaperones, DnaJB6 and DnaJB8 were the most potent suppressors of polyglutamine aggregation.
Interestingly, two splicing variants of DnaJB6 exist: one short and one long isoform that both are ubiquitously expressed (see below and Fig. 6). The longer message (ca 2470 nucleotides in length) encodes a protein of 326-amino acids, whereas the shorter transcript (approximately 1490 nucleotides in length) encodes a 241 amino acid protein (Hanai and Mashima, 2003). The two proteins differ in the C-terminal region. Due to the alternative splicing, the short isoform ends with a 10 amino acid motif KEQLLRLDNK not present in the long isoform whereas the long isoform has an extension of 85 amino acids. A major remarkable difference found between the two homologues is the existence of an experimentally verified nuclear localization signal in the long isoform not found in the short isoform which was cytosolic ((Hanai and Mashima, 2003) and figure 3A). Consistent with the fact that most of the polyglutamine protein resides in the cytosol (figure 1C), we found only the short and not the long isoform of DnaJB6 prevented polyglutamine aggregation (figure 3B-C). This shows that the effect of DnaJB6 requires compartimental co-localization and indicates that an interaction between the chaperone and the polyglutamine protein is required for its anti-aggregation effect.
It had been shown previously that DnaJB1 (alone or together with HSPA1A) is a strong suppressor of aggregation of polyglutamine proteins (see Rujano and Kampinga, 2007 for review), but in our model, this effect was rather minor. To test whether this is due to the length of the polyglutamine stretch used in our screen (119 glutamines), pEGFP-HDQ74 encoding a fragment of the exon-1 of the protein huntingtin with a shorter polyglutamine stretch (74 glutamines: HDQ74) was used in combination with some members of each family of chaperones. Again, none of the Hsp110, Hsp70 and DnaJA members tested suppressed HDQ74 aggregation, but now DnaJB1, like DnaJB6 and DnaJB8, indeed largely abolished it as detected by microscopy (data not shown), filter trap and western blotting (Fig. 4A-B). These results are consistent with other reports that DnaJB1 is indeed capable of suppressing aggregation of expanded polyglutamine stretches. However, in vitro assays with these relatively "short" polyglutamine expansions might be insufficiently discriminative to uncover more potent suppressors such as DnaJB6 and DnaJB8.

### Example 2

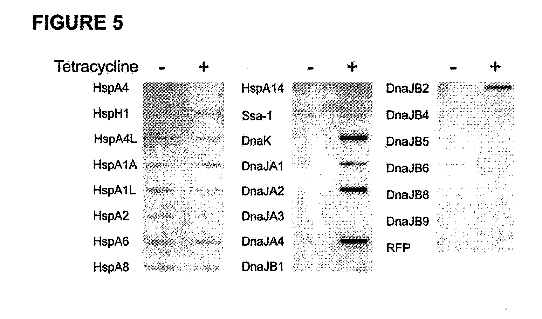

Previous studies had shown that some chaperones localize to polyglutamine aggregates which may titrate them away from their normal function (Chai et al., 1999; Cummings et al., 1998; Cummings et al., 2001; Jana et al., 2000; Hansson et al., 2003; Hay et al., 2004; Kobayashi et al., 2000; Stenoien et al., 1999; Wyttenbach et al., 2000; Sittler et al., 2001; Bailey et al., 2002; Warrick et al., 1999; Huen and Chan, 2005). To test this possibility in our model system, filter trap blots were probed with anti-V5 antibodies. Of all members of the ectopically expressed Hsp70 proteins, only the bacterial DnaK was found trapped on the aggregates in the filters. Also, the Hsp40 members DnaJA2, DnaJA4, DnaJB2 and to a lesser extent DnaJA1 were trapped (Fig. 5). Interestingly, specifically these proteins did not decrease the aggregation ofHDQ119 but rather enhanced it (see Fig. 1). Thus, some chaperones may not only get trapped in polyglutamine aggregates leading to a loss of their function, but by their interaction with misfolded proteins like HDQ119, they compete with the protective chaperones and as such enhance HDQ119 aggregation.

### Example 3

It has been shown previously, that the DnaJB6 short isoform is ubiquitously expressed in various human tissues and especially enriched in brain and retina (Hunter et al., 1999; Seki et al., 1999; Chuang et al., 2002a), whereas DnaJB6 long has been demonstrated to be widely expressed, although enriched in testis (Pei, 1999), but so far expression patterns of DnaJB8 have not been reported. In order to examine the expression profiles of these genes, we performed semiquantitative RT-PCR in cDNA prepared from total RNA of various cell lines and tissues (Fig. 6). DnaJB6 was found to be expressed rather ubiquitously, the short isoform always at somewhat higher levels than the long isoform consistent with previous reports (Hunter et al., 1999; Seki et al., 1999; Chuang et al., 2002a; Pei, 1999). DnaJB8 expression was very low: transcripts were detected only after 35 cycles in kidney, testis and fetal brain, and at very low level in all of the cell lines and other tissue samples except for colon.

### Example 4

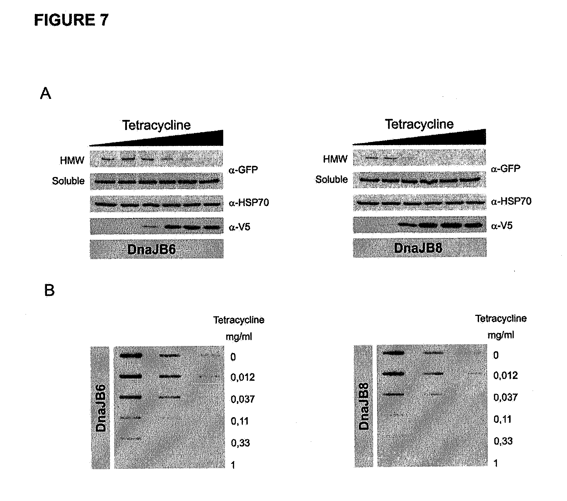

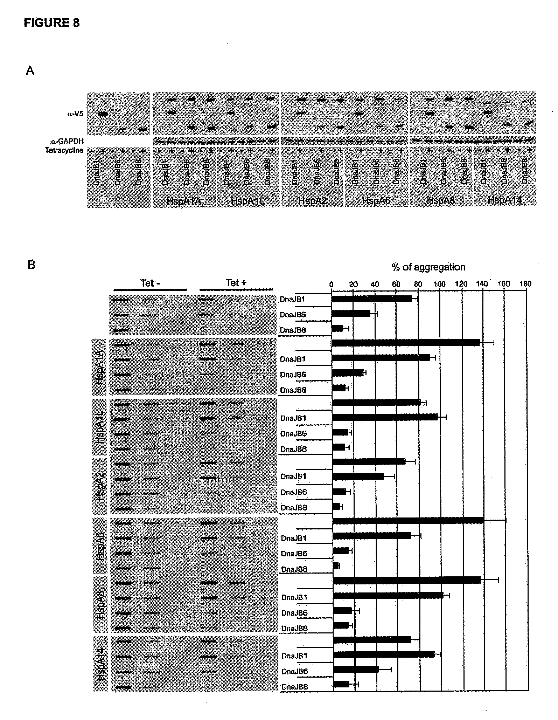

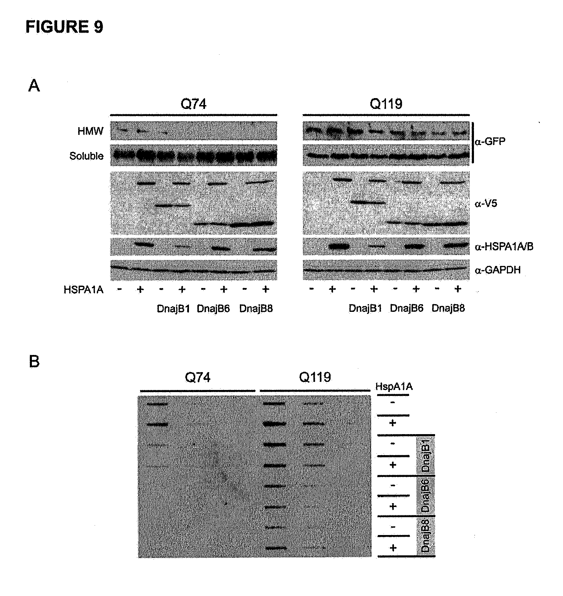

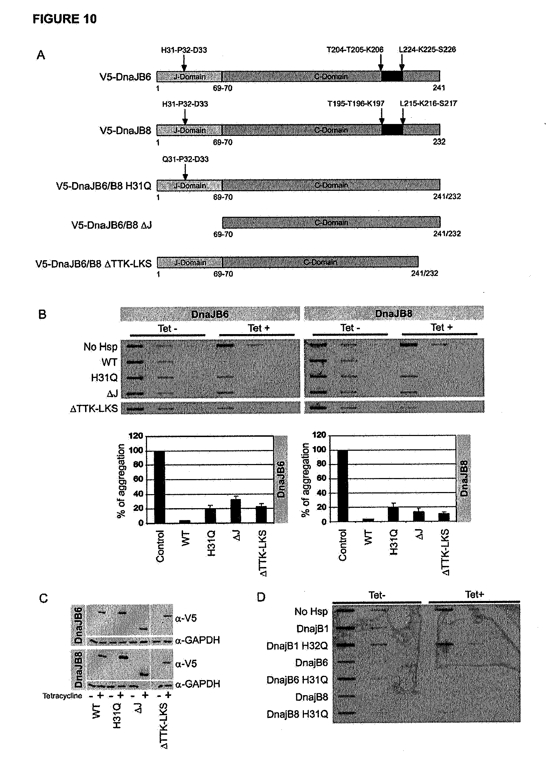

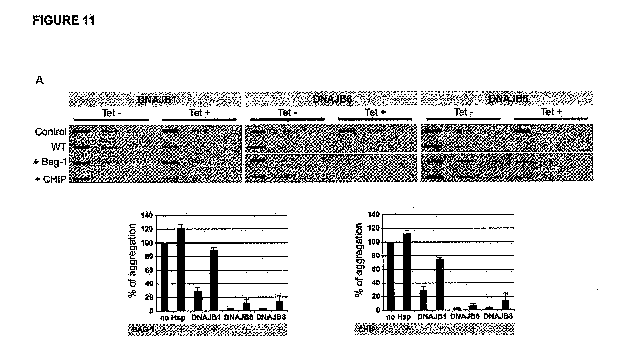

Hsp40 proteins are known as co-chaperones of Hsp70, and it is thought that especially members of the class B have little or no chaperone activity on their own. Indeed DnaJB 1 and HSPA1A were shown to act synergistically in preventing aggregation of e.g. androgen receptor (Bailey et al., 2002): To investigate whether DnaJB6 and DnaJB8 also interact with Hsp70 proteins to suppress polyglutamine aggregate formation, we first wanted to co-express DnaJB6 and DnaJB8 with diverse members of the Hsp70 family along with HDQ119. Because full expression of DnaJB6 or DnaJB8 resulted in almost complete inhibition of aggregation, we first modulated their respective concentrations and effectiveness using tetracycline-concentration dependent titrations (Fig. 7A-B). These data show that there is a tight correlation between levels of expression and extent of aggregation, indicating that DnaJB6 and DnaJB8 are functional in preventing aggregation at physiological relevant concentrations. From these experiments, it is concluded that DnaJB8 is more efficient than DnaJB6. Equal amounts of expression plasmids resulted in enhanced clearing of aggregated material by DnaJB8 for all tetracycline concentrations tested.
To test for putative synergy with Hsp70 members, we chose 0.074 µg/ml tetracycline: at this concentration DnaJB6 or DnaJB8 are clearly expressed but aggregation is not yet fully inhibited (Fig. 7B), allowing space for putative modulation. However, to our surprise co-expression of neither one of the Hsp70 members (Fig. 8A) could increase the anti-aggregation effect of DnaJB6 or DnaJB8 (Fig. 8B) nor enhance the fraction of cells without detectable aggregates (data not shown).
Since several members of the Hsp70 family are constitutively expressed, it remains possible that these levels are already sufficient to support the anti-aggregation action after overexpression of DnaJB6 and DnaJB8 alone. To test this option, the efficacy of these two molecules was assessed in 023 hamster fibroblasts which do not express endogenous HSPA1A and HspA1B under normal conditions ((Michels et al., 1997) and Fig. 9A). Overexpression of DnaJB6 or DnaJB8 (Fig. 9A) resulted in complete inhibition of HDQ74 aggregation (Fig. 9B), confirming that at least HspA1A is not required for the anti-aggregation ability of DnaJB6 or DnaJB8. However, it is still possible that one of the other Hsp70 family members is the interaction partner of DnaJB6 or DnaJB8 and is sufficient to support their effect. Therefore, we generated two DnaJB6 and DnaJB8 mutants (Fig. 10A and C), one lacking the entire J-domain (ΔJ) and one in which the histidine residue in the conserved HPD motif of the J-domain was substituted by a glutamine (H31Q). For prokaryotic DnaK-DnaJ or eukaryotic HSPA1A-DnaJB1 or HspA8-DnaJB1, these mutations abolish the possibility of interaction between these partners and lead to a loss of their functional cooperation (Kelley, 1998; Michels et al., 1999). However, both mutant proteins retained their ability to suppress HDQ119 aggregation, although slightly less efficient than the full length wildtype protein (Fig. 10B). This again contrasts the results for DnaJB1: here the H31Q mutation resulted in a near to complete loss of its anti-aggregation properties (Fig. 10D). Finally, we also tested whether Bag-1 or CHIP (Fig. 11A), two co-chaperones with antagonistic actions on Hsp70 activity as compared to Hsp40 proteins (Takayama et al., 1997; Nollen et al., 2000; Kampinga et al., 2003) would revert the effects of DnaJB6 or DnaJB8 on HDQ119 aggregation. Overexpression of both proteins alone had no effect on HDQ119 aggregation (Fig. 11). Furthermore, co-expression of either Bag-1 or CHIP did not significantly affected protection against HDQ119 aggregation as evoked by DnaJB6 or DnaJB8, but it did largely revert the effect of DnaJB1 (Fig. 11). Together these results show that, unlike DnaJB1, DnaJB6 and DnaJB8 suppress polyglutamine aggregation in a manner that does not require a direct classical interaction of their J-domain with the C-terminal ATPase domain of Hsp70 family members.

### Example 5

To test for putative functional domains responsible for the strong suppressive effect of DnaJB6 or DnaJB8 on HDQ119 aggregation, we searched for specific domains that are only found in DnaJB6 and DnaJB8 and that are not present in other Hsp40 family members (Fig. 10A; Figure 17)). A short, C-terminal sequence (TTKRIVENGQERVEVEEDGQLKS) was found to be unique for DnaJB6 and DnaJB8 and mutants lacking this short sequence were created (DnaJB6-ΔTTK-LKS and DnaJB8-ΔTTK-LKS, Fig. 10A and B) and analyzed for their anti-aggregation activity. The ΔTTK-LKS mutant proteins were still protective against HDQ119 aggregation, although not as efficient as the WT (Fig. 10C), indicating that this sequence is not crucial.

### Example 6

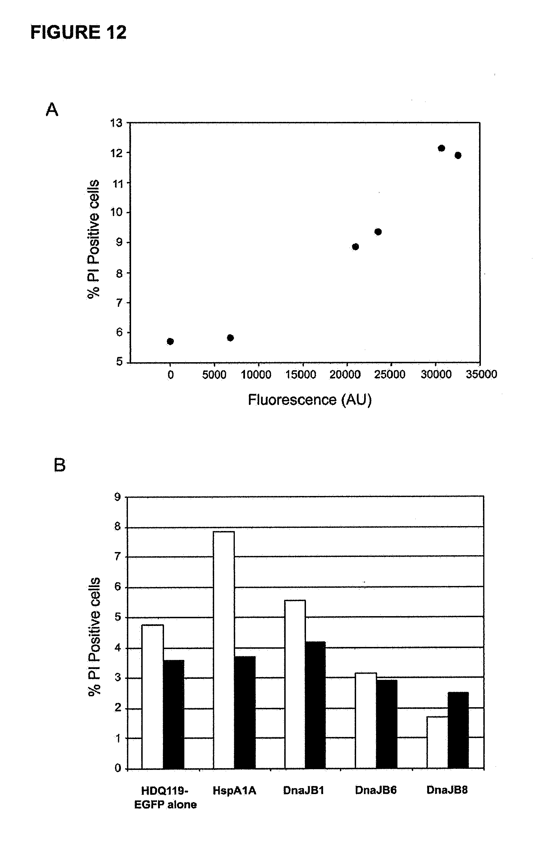

As can be seen in the various IF images (Fig. 1C) most of the polyglutamine aggregates were concentrated in single large aggregates adjacent to the nucleus, resembling aggresomes (Johnston et al., 1998; Kopito, 2000). Indeed, aggregated huntingtin in HEK293 cells localize adjacent to the centriole and is ensheated in a cage of vimentin (Rujano et al., 2006) consistent with their classification as aggresomes. Previous studies demonstrated that sorting misfolded proteins to aggresomes occurs when protein quality control has failed and as such serves as a (secondary) mechanism to protect cells against the more toxic aggregate intermediates (Johnston et al., 1998; Arrasate et al., 2004). Indeed, inhibition of aggresome formation was shown to increase cell toxicity (Taylor et al., 2003) and also other studies have recently suggested that intermediate sized aggregates might be more toxic than larger (dynamically assembled) aggregates (Behrends et al., 2006). As DnaJB8 nearly completely inhibited the formation of aggresomes, one could argue that this might result in increased toxicity rather than cellular protection. Therefore, we performed cell viability assays using a Propidium Iodide (PI) exclusion assay. Previously, we showed (Rujano et al., 2006) that HDQ119 expressed in HEK293 cells, mainly accumulates in aggresomes (80 % of cells with aggregates) and, consistent with that being cytoprotective, the percentage of cell death is low at early time points. However, increased expression of HDQ119-EYFP also causes an overload of this (final) rescue mechanism indicated by a progressive, albeit still low increase in the fraction of PI positive cells (Fig. 12A). In cells cotransfected with DnaJB8 the fraction of PI positive cells was strongly reduced (Fig. 12B), demonstrating that DnaJB8 inhibits polyglutamine aggregation in a manner that also results in protection against polyglutamine induced cytotoxicity. This shows that DnaJB8 acts at a stage prior to aggresome formation.

### Example 7

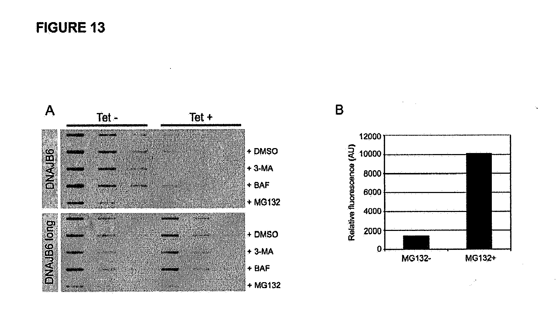

It is known that proteins that cannot be properly folded are usually targeted for degradation. Whilst decreases in the expression levels of the HDQ119 protein could not be detected in the former experiments after DnaJB8 expression, a conclusion cannot be drawn from this since the fraction of aggregated proteins is too small and its improved clearance may not be readily detectable as a decline in the bulk of the soluble protein. Therefore, we decided to check whether the inhibition of aggregation by DnaJB8 could be due to an increase in the degradation rate of the unfolded polyglutamine proteins by the proteasome or autophagy. To test this possibility, cells were cotransfected with HDQ119 and DnaJB8 and two hours after transfection, when expression of the chaperones was induced with the addition of tetracycline, cells were treated with the proteasome inhibitor MG132. Effectiveness of the drug on proteasomal inhibition was confirmed by the increase in expression of the GFPu reporter ((Bence et al., 2001) Fig. 13B). 16 hours after inhibitor treatment, the total level of polyglutamine aggregation (Fig. 13A) was still minimal in cells expressing DnaJB8, indicating that their protection was not linked with facilitated proteasomal degradation.
In a similar set-up, cells were treated with 3-methyladenine (3-MA) or Bafilomycin A1 (BAF), known inhibitors of (macro)-autophagy. Again, cells expressing DnaJB6 or DnaJB8 still showed virtually no aggregation (Fig. 13A and data not shown), demonstrating that also increased (macro)-autophagy mediated degradation cannot explain the absence of aggregates in the presence of DnaJB6 or DnaJB8. In fact, neither drug alone showed an enhancement of aggregation (data not shown) showing that such a large polyglutamine expansion (in the context of huntingtin) is a poor substrate for degradation via the proteasome or via (macro)-autophagy.

### Example 8

### Materials and methods

The muscle-specific expression vector pCac(A)2+ was made by replacing the CMV promoter of the Xenopus vector pCS2+ (Turner and Weintraub, 1994) with the 0.6-kb muscle-specific cardiac actin (Cac) promoter (Mohun et al., 1986). Plasmids pCac(A)2+DnaJB1, pCac(A)2+DnaJB6, pCac(A)2+DnaJB8, and pCac-Q119-YFP were made by cloning the NotI (Klenow filled-in) - HindIII fragments of, respectively, pcDNA5-FRT-TO-V5-DnaJB1, pcDNA5-FRT-TO-V5-DnaJB6, pcDNA5-FRT-TO-V5-DnaJB8, and pQ119-eYFP into the HindIII and StuI sites of pCac(A)2+.

Expression cassettes consisting of the Cac promoter, polyQ/DnaJ protein coding region, and SV40 polyadenylation signal were excised from the pCS2+ vector backbone using SalI and NotI, separated via agarose gel electrophoresis and recovered from the agarose slices using the GFX gel band purification kit (Amersham, UK). Transgenesis of Xenopus laevis was performed according to Kroll and Amaya (1996), with modifications according to Sparrow et al. (2000). In summary, 250,000 sperm nuclei were mixed with ~250 ng expression cassette (125 ng Q119-YFP and 125 ng DnaJ*), incubated for 15 min at RT and diluted in 500 µl sperm dilution buffer (250 mM sucrose, 75 mM KCl, 0.5 mM spermidine trihydrochloride, 0,2 mM spermidine tetrahydrochloride, 5 mM MgCl2, pH 7.4). Eggs were dejellied in 2% cystein/1 x MMR (1 x MMR: 0.1 M NaCl, 0.02 M KCl, 0.01 M MgC12, 0.015 M CaCl2 en 0.5 M HEPES pH 7.5), transferred to 6% Ficoll/0.4 x MMR and injected with 10 nl of the diluted nuclei/DNA mixture at 17°C. Embryos at the 4-cell stage were transferred to 6% Ficoll/0.1 x MMR and incubated O/N at 17°C. Gastrulae (Nieuwkoop stage 12) were transferred to 0.1 x MMR and incubated at 22°C. YFP-positive tadpoles were immobilized with MS-222 and photographed using an MZ FLIII fluorescence stereomicroscope provided with a DC200 camera (Leica microsystems, Switzerland).

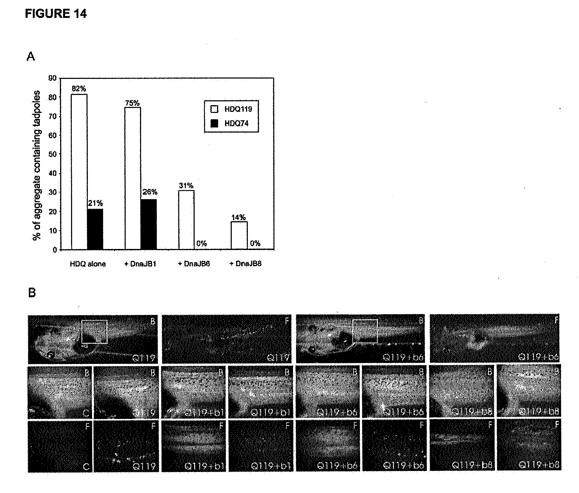

Although the precise mechanism by which DnaJB8 modulates polyglutamine aggregation and toxicity in our cell model is not clear yet, we decided to test whether this protective effect would also be seen in an animal model. As a first method to test whether DnaJB8-mediated inhibition of aggregation also holds true *in vivo,* we employed a Xenopus laevis tadpole's model. Expression of EYFP-tagged polyglutamine containing huntingtin (HDQ119-EYFP) under control of a muscle-specific promoter into embryos leads to developmental-related mortality, and those adults that did survive showed many inclusions throughout the body (Fig. 14). EGFP-tagged, non-expanded huntingtin or GFP alone was non toxic and resulted in diffuse staining patterns (data not shown). Co-expression with DnaJB8 nearly completely annihilated aggregation and also reduced development-related death (Fig 14). In contrast, co-expression of DnaJB1 did not rescue HDQ119 aggregation and related toxicity. These data are consistent with our cell culture data and show that DnaJB8 also protect in vivo. Moreover, it shows that the anti-aggregation properties of DnaJB8 is conserved in vertebrates, demonstrating a highly evolutionary conserved mechanism.

### Example 9

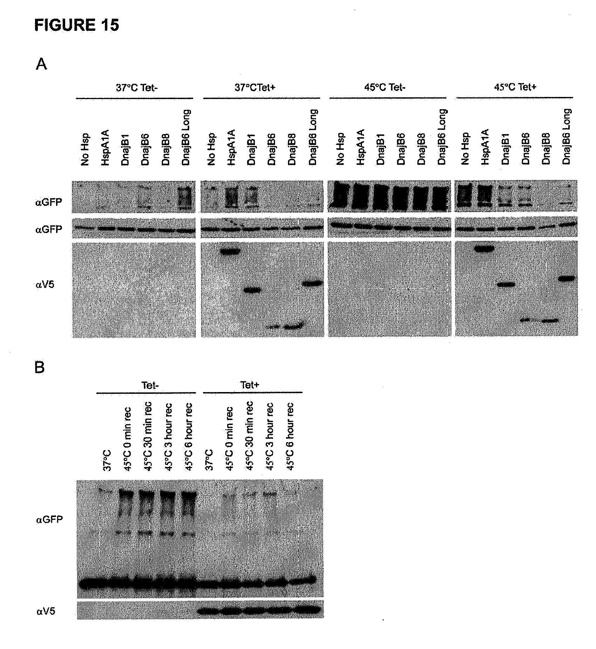

DnajB6 and DnaJB8 block aggregation of non-polyQ substrates. Luciferase was cotransfected with various chaperones. The day after, cells were incubated at 37°C or 45°C for 30 minutes, lysed and analyzed on western blot. Aggregated luciferase is detected in the stacking gel. Non aggregated luciferase is detected as a single band in the running gel. The results are shown in Figure 15. It is clear that aggregation of luciferase, which is a non-polyQ substrate, is also counteracted by DnaJB6 and DnaJB8.

### Brief description of the drawings

**Figure 1****. Modulation of polyglutamine aggregation by overexpression of (V5-tagged) molecular chaperones.** Cells were cotransfected with HDQ119-EYFP and tetracycline inducible, V5-tagged members of Hsp110, Hsp70 and Hsp40 families of molecular chaperones. 2h after transfection, the expression of the chaperones was induced in one of two identical transfections by adding tetracycline containing medium to the cells.
   (A) Western blot analysis of cell extracts prepared 24h after transfection. High molecular weight (HMW) aggregates trapped in the stacking gel and soluble HDQ119-EYFP, were assessed with anti-GFP antibodies. Coexpression of molecular chaperones after tetracycline induction was verified with anti-V5 antibodies.
   (B) Filter trap assay in samples without and with overexpression of the chaperones (-/+ tetracycline) prepared 24h after cotransfection. Samples were slot-blotted at three different concentrations (10, 2 and 0.4 □g of protein/slot) in cellulose -acetate membranes and probed with anti-GFP antibody. Intensity of the bands was quantified by densitometry and % of polyglutamine aggregation was calculated for each concentration of protein by setting the off situation (-Tet) to 100%. Bars indicate standard error of the mean of 3-6 different measurements.
   (C) Representative confocal images of cells cotransfected with HDQ-EYFP (light grey) and V5-tagged members of Hsp110, Hsp70 and Hsp40 families of molecular chaperones immunostained with anti-V5 antibody (grey). DNA is counterstained with DAPI (dark grey). Bars represent 30 □m.
**Figure 2****: Modulation of polyglutamine aggregation by overexpression of (non-tagged) molecular chaperones.** Cells were cotransfected with HDQ119-EYFP and tetracycline inducible, members of Hsp110, Hsp70 and Hsp40 families of molecular chaperones. 2h after transfection, the expression of the chaperones was induced in one of two identical transfections by adding tetracycline containing medium to the cells.
   (A) Filter trap assay in samples without and with overexpression of the chaperones (-/+ tetracycline) prepared 24h after cotransfection. Samples were slot-blotted at three different concentrations (10, 2 and 0.4 □g of protein/slot) in cellulose -acetate membranes and probed with anti-GFP antibody. Intensity of the bands was quantified by densitometry and % of polyglutamine aggregation was calculated for each concentration of protein by setting the off situation (-Tet) to 100%. Bars indicate standard error of the mean of 3-6 different measurements.
   (B) Western blot analysis of cell extracts prepared 24h after transfection. High molecular weight (HMW) aggregates trapped in the stacking gel and soluble HDQ119-EYFP, were assessed with anti-GFP antibodies. Due to lack of specific antibodies expression of molecular chaperones after tetracycline induction was not verified.
**Figure 3****: Only the short and not the long isoform of DnaJB6 prevented polyglutamine aggregation.** Cells were cotransfected with HDQ119-EYFP and DnaJB6 short or long isoform. 2h after transfection, the expression of the chaperones was induced in one of two identical transfections by adding tetracycline containing medium to the cells.
   (A) Sequence alignment of the long (DnaJB6_A) and short (DnaJB6_B) isoforms of DnaJB6. The putative nuclear localization signal (NLS) in the long isoform is underlined.
   (B) Western blot analysis of cell extracts prepared 24h after transfection. High molecular weight (HMW) aggregates trapped in the stacking gel and soluble HDQ119-EYFP, were assessed with anti-GFP antibodies. Coexpression of DnaJB6 short and DnaJB6 long after tetracycline induction was verified with anti-V5 antibodies.
   (C) Filter trap assay in samples without and with overexpression of DnaJB6 short and DnaJB6 long (-/+ tetracycline) prepared 24h after cotransfection. Samples were slot-blotted at three different concentrations (10, 2 and 0.4 □g of protein/slot) in cellulose-acetate membranes and probed with anti-GFP antibody.
**Figure 4****: DnaJB1 is a strong suppressor of aggregation of shorter polyglutamine stretches.** Cells were cotransfected with EYFP-HDQ74 and some members ofHsp110 (HspA4), Hsp70 (HspA1L, HspA6 and HspA8) and Hsp40 (DnaJA1, DnaJB1, DnaJB6 and DnaJB8) families of molecular chaperones. 2h after transfection, the expression of the chaperones was induced in one of two identical transfections by adding tetracycline containing medium to the cells.
   (A) Western blot analysis of cell extracts prepared 24h after transfection. High molecular weight (HMW) aggregates trapped in the stacking gel and soluble EYFP-HDQ74, were assessed with anti-GFP antibodies. Coexpression of the chaperones after tetracycline induction was verified with anti-V5 antibodies. The GAPDH housekeeping protein was used as loading control.
   (B) Filter trap assay in samples without and with overexpression of the chaperones (-/+ tetracycline) prepared 24h after cotransfection. Samples were slot-blotted at three different concentrations (10, 2 and 0.4 □g of protein/slot) in cellulose - acetate membranes and probed with anti-GFP antibody. Intensity of the bands was quantified by densitometry and % of polyglutamine aggregation was calculated for each concentration of protein by setting the off situation (-Tet) to 100%.
**Figure 5****: Some members of the Hsp70 and Hsp40 family of molecular chaperones are trapped in HDQ119 aggregates.** Filter trap assay in samples without and with overexpression of the chaperones (-/+ tetracycline) prepared 24h after cotransfection. Samples were slot-blotted at a concentration of 10 □g of protein/slot in cellulose-acetate membranes and probed with anti-V5 antibody.
**Figure 6****: Cell line and tissue expression patterns of DnaJB6 short and long isoforms and DnaJB8.**
   (A) Name and origin of the different cell lines assayed for DnaJB6 short and long isoforms and DnaJB8 expression.
   (B) RT-PCR performed on cDNA prepared from total RNA of various cell lines. Samples were taken after 20, 25 and 30 cycles and analyzed in a 0.8 % agarose gel. GAPDH was used as control housekeeping gene.
   (C) RT-PCR performed on cDNA prepared from total RNA of various human tissues. Samples were taken after 20, 25 and 30 cycles for DnaJB6 short and long isoforms and 20, 25, 30 and 35 cycles for DnaJB8 and analyzed in a 0.8 % agarose gel. GAPDH was used as control housekeeping gene.
**Figure 7****: DnaJB6 and DnaJB8 are functional in preventing aggregation at physiological relevant concentrations.** Cells were cotransfected with HDQ119-EYFP and DnaJB6 and DnaJB8. Tetracycline-concentration dependent titration of DnaJB6 and DnaJB8 expression was performed 2h after transfection, by adding increasing amounts of tetracycline to the growth medium.
   (A) Western blot analysis of cell extracts prepared 24h after transfection. High molecular weight (HMW) aggregates trapped in the stacking gel and soluble HDQ119-EYFP, were assessed with anti-GFP antibodies. Increased expression of DnaJB6 and DnaJB8 after tetracycline induction was verified with anti-V5 antibodies.
   (B) Filter trap assay in samples with tetracycline-concentration dependent titration of DnaJB6 and DnaJB8 expression prepared 24h after cotransfection. Samples were slot-blotted at three different concentrations (10, 2 and 0.4 □g of protein/slot) in cellulose-acetate membranes and probed with anti-GFP antibody.
**Figure 8****: Hsp70 co-expression does not enhance the suppressive effects of DnaJB6 and DnaJB8 on aggregation of HDQ119.** Cells were cotransfected with HDQ119-EYFP and combinations of each Hsp70 family member with DnaJB1, DnaJB6 or DnaJB8. 2h after transfection, the expression of the chaperones was induced in one of two identical transfections by adding a suboptimal amount of tetracycline (0.074 □g/ml) to the growth medium.
   (A) Western blot analysis of cell extracts prepared 24h after transfection. Coexpression of the chaperones after tetracycline induction was verified with anti-V5 antibodies. GAPDH housekeeping protein was used as loading control.
   (B) Filter trap assay in samples without and with overexpression of the chaperones (-/+ tetracycline) prepared 24h after cotransfection. Samples were slot-blotted at three different concentrations (10, 2 and 0.4 □g of protein/slot) in cellulose -acetate membranes and probed with anti-GFP antibody. Intensity of the bands was quantified by densitometry and % of polyglutamine aggregation was calculated for each concentration of protein by setting the off situation (-Tet) to 100%. Bars indicate standard error of the mean of 3-6 different measurements.
**Figure 9****: DnaJB6 and DnaJB8 suppress aggregation of HDQ74 and HDQ119 in 023 hamster fibroblasts which do not express endogenous HspA1A and HspA1B under normal growth conditions.** Cells were cotransfected with EGFP-HDQ74 or HDQ119-EYFP and DnaJB1, DnaJB6 or DnaJB8 with and without HspA1A. Expression of HspA1A and the DnaJB proteins in these cells is driven by the constitutively active CMV promoter.
   (A) Western blot analysis of cell extracts prepared 24h after transfection. High molecular weight (HMW) aggregates trapped in the stacking gel and soluble HDQ119-EYFP, were assessed with anti-GFP antibodies. Coexpression of molecular chaperones after tetracycline induction was verified with anti-V5 antibodies. Expression of endogenous HspA1A. and HspA1B was verified with anti-Hsp70 antibodies. Note that when no ectopically added, these chaperones are not expressed or induced in these cells.
   (B) Filter trap assay in samples prepared 24h after cotransfection. Samples were slot-blotted at three different concentrations (10, 2 and 0.4 □g of protein/slot) in cellulose-acetate membranes and probed with anti-GFP antibody.
**Figure 10****: Inhibition of aggregation by DnaJB6 and DnaJB8 is not lost after mutating or deleting the J domain and does not require the C-terminal conserved motif TTKRIVENGQERVEVEEDGQLKS.** Cells were cotransfected with HDQ119-EYFP and either wildtype DnaJB6 or DnaJB8 (WT) or the mutants H31Q, □□J and □TTK-LKS. 2h after transfection, the expression of the chaperones and mutants was induced in one of two identical transfections by adding tetracycline containing medium to the cells.
   (A) Schematic representation of WT DnaJB6 and DnaJB8, a mutant in which the histidine residue in the conserved HPD motif of the J-domain was substituted by a glutamine (H31Q), a mutant lacking the entire J-domain (□J) and a mutant lacking the C-terminal unique motif (□TTK-LKS).
   (B) Filter trap assay in samples without and with overexpression of WT and mutants (□J, H31Q and □TTK-LKS) of DnaJB6 and DnaJB8 (-/+ tetracycline) prepared 24h after cotransfection. Samples were slot-blotted at three different concentrations (10, 2 and 0.4 □g of protein/slot) in cellulose-acetate membranes and probed with anti-GFP antibody. Intensity of the bands was quantified by densitometry and % of polyglutamine aggregation was calculated for each concentration of protein by setting the off situation (-Tet) to 100%. Bars indicate standard error of the mean of 3-6 different measurements.
   (C) Western blot analysis of cell extracts prepared 24h after transfection. Expression of WT and mutants (□J, H31Q and □TTK-LKS) of DnaJB6 and DnaJB8 after tetracycline induction was verified with anti-V5 antibodies. GAPDH housekeeping protein was used as loading control.
   (D) Comparative filter trap assay in samples without and with overexpression of WT, H32Q mutant of DnaJB1, and H31Q mutants of DnaJB6 and DnaJB8 (-/+ tetracycline) prepared 24h after transfection. Samples were slot-blotted at three different concentrations (10, 2 and 0.4 □g of protein/slot) in cellulose-acetate membranes and probed with anti-GFP antibody.
**Figure 11****: Inhibition of aggregation by DnaJB1 but not DnaJB6 and DnaJB8 is reverted by coexpression of Bag-1 or CHIP.** Cells were cotransfected with HDQ119-EYFP and either DnaJB6 or DnaJB8 and Bag-1 or CHIP. Expression of the chaperones and cochaperones was induced 2h after transfection in one of two identical transfections by adding tetracycline containing medium to the cells.
   (A) Filter trap assay in samples without and with overexpression of DnaJB1, DnaJB6 and DnaJB8 and coexpression of Bag-1 and CHIP (-/+ tetracycline) prepared 24h after cotransfection. Samples were slot-blotted at three different concentrations (10, 2 and 0.4 □g of protein/slot) in cellulose-acetate membranes and probed with anti-GFP antibody. Intensity of the bands was quantified by densitometry and % of polyglutamine aggregation was calculated for each concentration of protein by setting the off situation (-Tet) to 100%. Bars indicate standard error of the mean of 3-6 different measurements.
**Figure 12****: Suppression of aggregation by DnaJB6 and DnaJB8** is **paralelled by reduction in polyglutamine-induced cytotoxicity.** Cells were cotransfected with HDQ119-EYFP and DnaJB6 or DnaJB8. Expression of the chaperones and cochaperones was induced 2h after transfection in one of two identical transfections by adding tetracycline containing medium to the cells. Cell death was measured by Propidium Iodide (PI) incorporation after 48 and 96 hours by flow cytometry counting 10.000 cells in each group. Percentage of GFP positive cells that incorporate the dye (GFP + / PI +) was used as the percentage of dead cells.
   (A) Exponential accumulation of PI positive cells in cultures transfected with increased amounts of HDQ119-EYFP. The HDQ119-EYFP content in cells was measured by fluorometry and is expressed as arbitrary units (AU).
   (B) Percentage of PI positive cells or dead cells in the cultures after 48 and 96 hours of expression of HDQ119-EYFP alone or together with HspA1A, DnaJB1, DnaJB6 or DnaJB8.
**Figure 13****: DnaJB6 and DnaJB8 modulation of polyglutamine aggregation is not related to proteasomal mediated degradation or macroautophagy.** Cells were cotransfected with HDQ119-EYFP and DnaJB6 short and long isoforms or DnaJB8 (not shown). Expression of the chaperones was induced 2h after transfection in one of two identical transfections by adding tetracycline containing medium to the cells. Inhibitors of macroautophagy (3-MA and BAF) and the proteasome (MG132) were also added 2h after transfection and the treatment was performed for 16 hours.
   (A) Filter trap assay in samples without and with overexpression of DnaJB6 short and long isoforms (-/+ tetracycline) and treated with the macroautophagy inhibitors 3-MA and BAF or the proteasome inhibitor MG132, prepared 24h after transfection. Samples were slot-blotted at three different concentrations (10, 2 and 0.4 □g of protein/slot) in cellulose-acetate membranes and probed with anti-GFP antibody.
   (B) GFPu accumulation in the presence of the proteasome inhibitor MG132. Cells transfected with the ubiquitin-proteasome system reporter GFPu, were treated with the proteasomal inhibitor MG132 for 16 hours. Accumulation of the reporter was measured by fluorometry.
**Figure 14****: DnaJB6 and DnaJB8 modulation of polyglutamine aggregation in *Xenopus laevis* tadpoles.** Transgenic HDQ119-EYFP/HDQ74-EGFP positive tadpoles were immobilized with MS-222 and analyzed by fluorescence microscopy.
   (A) Percentage (%) of aggregate containing tadpoles when polyglutamine containing proteins are expressed alone or in combination with DnaJB1, DnaJB6 or DnaJB8.
   (B) Representative fluorescence images of tadpoles expressing HDQ119-EYFP (green) alone or in combination with DnaJB1, DnaJB6 or DnaJB8.
**Figure 15****: DnajB6 and DnaJB8 block aggregation of non-polyQ substrates.**
**Figure 16****: DnaJB8 paralogs**
**Figure 17****: DnaJB8 paralogs**

### References

Arrasate,M., Mitra,S., Schweitzer,E.S., Segal,M.R., and Finkbeiner,S. (2004). Inclusion body formation reduces levels of mutant huntingtin and the risk of neuronal death. Nature 431, 805-810.
Bailey,C.K., Andriola,I.F.M., Kampinga,H.H., and Merry,D.E. (2002). Molecular chaperones enhance the degradation of expanded polyglutamine repeat androgen receptor in a cellular model of spinal and bulbar muscular atrophy. Hum. Mol. Genet. 11, 515-523.
Behrends,C., Langer, C.A., Boteva,R., Bottcher,U.M., Stemp,M.J., Schaffar,G., Rao,B.V., Giese,A., Kretzschmar,H., Siegers,K., and Hartl,F.U. (2006). Chaperonin TRiC promotes the assembly of polyQ expansion proteins into nontoxic oligomers. Mol. Cell 23, 887-897.
Bence,N.F., Sampat,R.M., and Kopito,R.R. (2001). Impairment of the ubiquitin-proteasome system by protein aggregation. Science 292, 1552-1555.
Carra,S., Sivilotti,M., Chavez Zobel,A.T., Lambert,H., and Landry,J. (2005). HspB8, a small heat shock protein mutated in human neuromuscular disorders, has in vivo chaperone activity in cultured cells. Hum. Mol. Genet. 14, 1659-1669.
Chai,Y.H., Koppenhafer,S.L., Bonini,N.M., and Paulson,H.L. (1999). Analysis of the role of heat shock protein (Hsp) molecular chaperones in polyglutamine disease. J. Neurosci. 19, 10338-10347.
Cheetham,M.E. and Caplan,A.J. (1998). Structure, function and evolution of DnaJ: conservation and adaptation of chaperone function. Cell Stress & Chaperones 3, 28-36.
Chuang,J.Z., Zhou,H., Zhu,M., Li,S.H., Li,X.J., and Sung,C.H. (2002a). Characterization of a brain-enriched chaperone, MRJ, that inhibits Huntingtin aggregation and toxicity independently. J. Biol. Chem. 277, 19831-19838.
Cummings;C.J., Mancini,M.A., Antalffy,B., DeFranco,D.B., Orr,H.T., and Zoghbi,H.Y. (1998). Chaperone suppression of aggregation and altered subcellular proteasome localization imply protein misfolding in SCA1. Nat. Gen. 19, 148-154.
Cummings,C.J., Sun,Y., Opal,P., Antalffy,B., Mestril,R., Orr,H.T., Dillmann,W.H., and Zoghbi,H.Y. (2001). Over-expression of inducible HSP70 chaperone suppresses neuropathology and improves motor function in SCA1 mice. Hum. Mol. Genet. 10, 1511-1518.
Fernandez-Funez,P., Nino-Rosales,M.L., de Gouyon,B., She,W.C., Luchak,J.M., Martinez,P., Turiegano,E., Benito,J., Capovilla,M., Skinner,P.J., McCall,A., Canal,I., Orr,H.T., Zoghbi,H.Y., and Botas,J. (2000). Identification of genes that modify ataxin-1-induced neurodegeneration. Nature 408, 101-106.
Frydman,J. (2001). Folding of newly translated proteins in vivo: the role of molecular chaperones. Annu. Rev. Biochem. 70, 603-647.
Gurbuxani et al. Oncogene 20: 7478-7485 (2001)).
Hanai,R. and Mashima,K. (2003). Characterization of two isoforms of a human DnaJ homologue, HSJ2. Mol. Biol. Rep. 30, 149-153.
Hansson,O., Nylandsted,J., Castilho,R.F., Leist,M., Jaattela,M., and Brundin,P. (2003). Overexpression of heat shock protein 70 in R6/2 Huntington's disease mice has only modest effects on disease progression. Brain Research 970, 47-57.
Hartl,F.U. and Hayer-Hartl,M. (2002). Protein folding - Molecular chaperones in the cytosol: from nascent chain to folded protein. Science 295, 1852-1858.
Hay,D.G., Sathasivam,K., Tobaben,S., Stahl,B., Marber,M., Mestril,R., Mahal,A., Smith,D.L., Woodman,B., and Bates, G.P. (2004). Progressive decrease in chaperone protein levels in a mouse model of Huntington's disease and induction of stress proteins as a therapeutic approach. Hum. Mol. Genet. 13, 1389-1405.
Holmberg,C.I., Staniszewski,K.E., Mensah,K.N., Matouschek,A., and Morimoto,R.I. (2004). Inefficient degradation of truncated polyglutamine proteins by the proteasome. Embo Journal 23, 4307-4318.
Huen,N.Y. and Chan,H.Y. (2005). Dynamic regulation of molecular chaperone gene expression in polyglutamine disease. Biochem. Biophys. Res. Commun. 334, 1074-1084.
Hunter,P.J., Swanson,B.J., Haendel,M.A., Lyons,G.E., and Cross,J.C. (1999). Mrj encodes a DnaJ-related co-chaperone that is essential for murine placental development. Development 126, 1247-1258.
Jana,N.R., Tanaka,M., Wang,G.H., and Nukina,N. (2000). Polyglutamine length-dependent interaction of Hsp40 and Hsp70 family chaperones with truncated N-terminal huntingtin: their role in suppression of aggregation and cellular toxicity. Hum. Mol. Genet. 9, 2009-2018.
Johnston,J.A., Ward,C.W., and Kopito,R.R. (1998). Aggresomes: A cellular response to misfolded proteins. J. Cell Biol. 143, 1883-1898.
Kampinga,H.H., Kanon,B., Salomons,F.A., Kabakov,A.E., and Patterson,C. (2003). Overexpression of the cochaperone CHIP enhances Hsp70-dependent folding activity in mammalian cells. Mol. Cell Biol. 23, 4948-4958.
Kazemi-Esfarjani,P. and Benzer,S. (2000). Genetic suppression of polyglutamine toxicity in Drosophila. Science 287, 1837-1840.
Kelley,W.L. (1998). The J-domain family and the recruitment of chaperone power. Trends in Biochemical Sciences 23,222-227.
Kobayashi,Y., Kume,A., Li,M., Doyu,M., Hata,M., Ohtsuka,K., and Sobue,G. (2000). Chaperones Hsp70 and Hsp40 suppress aggregate formation and apoptosis in cultured neuronal cells expressing truncated androgen receptor protein with expanded polyglutamine tract. J. Biol. Chem. 275, 8772-8778.
Kopito,R.R. (2000). Aggresomes, inclusion bodies and protein aggregation. Trends Cell Biol. 10, 524-530.
Kroll,K.L. and Amaya,E. (1996). Transgenic Xenopus embryos from sperm nuclear transplantations reveal FGF signaling requirements during gastrulation. Development 122, 3173-3183.
Lipinski et al. (1997) Adv Drug Deliv Rev 23: 3-25).
Michels,A.A., Kanon,B., Bensaude,O., and Kampinga,H.H. (1999). Heat shock protein (Hsp) 40 mutants inhibit Hsp70 in mammalian cells. J. Biol. Chem. 274, 36757-36763.
Michels,A.A., Kanon,B., Konings,A.W.T., Ohtsuka,K., Bensaude,O., and Kampinga,H.H. (1997). Hsp70 and Hsp40 chaperone activities in the cytoplasm and the nucleus of mammalian cells. J. Biol. Chem. 272, 33283-33289.
Mohun,T.J., Garrett,N., and Gurdon,J.B. (1986). Upstream sequences required for tissue-specific activation of the cardiac actin gene in Xenopus laevis embryos. EMBO J. 5, 3185-3193.
Nollen,E.A.A., Brunsting,J.F., Song,J.H., Kampinga,H.H., and Morimoto,R.I. (2000). Bag1 functions in vivo as a negative regulator of Hsp70 chaperone activity. Mol. Cell Biol. 20, 1083-1088.
Nollen,E.A.A., Garcia,S.M., van Haaften,G., Kim,S., Chavez,A., Morimoto,R.I., and Plasterk,R.H.A. (2004). Genome-wide RNA interference screen identifies previously undescribed regulators of polyglutamine aggregation. Proceedings of the National Academy of Sciences of the United States of America 101; 6403-6408.
Pei,L. (1999). Pituitary tumor-transforming gene protein associates with ribosomal protein S10 and a novel human homologue of DnaJ in testicular cells. J. Biol. Chem. 274, 3151-3158.
Rujano,M.A., Bosveld,F., Salomons,F.A., Dijk,F., van Waarde,M.A., van der Want,J.J., de Vos,R.A., Brunt,E.R., Sibon,O.C., and Kampinga,H.H. (2006). Polarised Asymmetric Inheritance of Accumulated Protein Damage in Higher Eukaryotes. PLoS. Biol. 4, e417.
Rujano,M.A. and Kampinga,H.H. (2007). The HSP70 chaperone machine as guardian of the proteome: Implications for protein misfolding diseases. In Heat Shock Proteins in Biology and Medicine, J.Radons and G.Multhoff, eds. Research Signpost).
Segal et al. (1999) PNAS 96: 2758-2763
Seki,N., Hattori,A., Hayashi,A., Kozuma,S., Miyajima,N., and Saito,T. (1999). Cloning, tissue expression, and chromosomal assignment of human MRJ gene for a member of the DNAJ protein family. J. Hum. Genet. 44, 185-189.
Sittler,A., Lurz,R., Lueder,G., Priller,J., Hayer-Hartl,M.K., Hartl,F.U., Lehrach,H., and Wanker,E.E. (2001). Geldanamycin activates a heat shock response and inhibits huntingtin aggregation in a cell culture model of Huntington's disease. Hum. Mol. Genet. 10, 1307-1315.
Sparrow,D.B., Latinkic,B. , and Mohun,T.J. (2000). A simplified method of generating transgenic Xenopus. Nucleic Acids Res. 28, E12.
Stenoien,D.L., Cummings,C.J., Adams,H.P., Mancini,M.G., Patel,K., DeMartino,G.N., Marcelli,M., Weigel,N.L., and Mancini,M.A. (1999). Polyglutamine-expanded androgen receptors form aggregates that sequester heat shock proteins, proteasome components and SRC-1, and are suppressed by the HDJ-2 chaperone. Hum. Mol. Genet. 8, 731-741.
Takayama,S., Bimston,D.N., Matsuzawa,S., Freeman,B.C., AimeSempe,C., Xie,Z.H., Morimoto,R.I., and Reed,J.C. (1997). BAG-1 modulates the chaperone activity of Hsp70/Hsc70. Embo Journal 16, 4887-4896.
Taylor,J.P., Tanaka,F., Robitschek,J., Sandoval,C.M., Taye,A., Markovic-Plese,S., and Fischbeck,K.H. (2003). Aggresomes protect cells by enhancing the degradation of toxic polyglutamine-containing protein. Hum. Mol. Genet. 12, 749-757.
Turner,D.L. and Weintraub,H. (1994). Expression of achaete-scute homolog 3 in Xenopus embryos converts ectodermal cells to a neural fate. Genes Dev. 8, 1434-1447.
Venkatraman,P., Wetzel,R., Tanaka,M., Nukina,N., and Goldberg,A.L. (2004). Eukaryotic proteasomes cannot digest polyglutamine sequences and release them during degradation of polyglutamine-containing proteins. Mol. Cell 14, 95-104.
Warrick,J.M., Chan,H.Y.E., Gray-Board, Chai,Y.H., Paulson,H.L., and Bonini,N.M. (1999). Suppression of polyglutamine-mediated neurodegeneration in Drosophila by the molecular chaperone HSP70. Nat. Gen. 23, 425-428.
Wyttenbach,A., Carmichael,J., Swartz,J., Furlong,R.A., Narain,Y., Rankin,J., and Rubinsztein,D.C. (2000). Effects of heat shock, heat shock protein 40 (HDJ-2), and proteasome inhibition on protein aggregation in cellular models of Huntington's disease. Proc. Natl. Acad. Sci. U. S. A 97, 2898-2903 (Wood et al. (2003) Neuropathology and Applied Neurobiology 29: 529-545).

## Claims

1. Use of a substance that is capable of enhancing the amount and/or activity of DnaJB8, or a functional part, derivative and/or analogue thereof, for the preparation of a medicament for at least in part treating and/or preventing a disorder associated with protein aggregation.

2. Use according to claim 1, whereby said disorder is associated with polyglutamine-mediated protein aggregation.

3. Use according to claim 1 or claim 2, whereby said disorder is selected from Huntington's disease, Dentatorubral-pallidoluysian atrophy (DRPLA), X-linked spinal and bulbar muscular atrophy (SBMA) and/or spinocerebellar ataxias (SCA)

4. Use of a substance that is capable of enhancing the amount and/or activity of DnaJB8, or a functional part, derivative and/or analogue thereof, for preventing aggregation of a protein.

5. Use according to claim 4, wherein aggregation of a protein is prevented in a mammalian cell.

6. Use according to claim 4 or claim 5, whereby preventing aggregation of a protein results in enhanced recovery of said protein.

7. Use according to any one of claims 1-6, whereby said substance comprises a small molecule compound.

8. Use according to any one of claims 1-7, whereby said substance comprises DnaJB8 protein, or a functional part, derivative and/or analogue thereof.

9. Use according to any one of claims 1-8, whereby said substance comprises the amino acid sequence
GAFSAGFGEFPAFMEAFSSFNMLGCSGGSHTTFSSTSFGGSSSGSSGFKSVMS STEMINGHKVTTKRIVENGQERVEVEEDGQLKSVTVNGKEQLKWMDSK.

10. Use according to any of claims 1-9, whereby said substance comprises a nucleic acid encoding a DnaJB8 protein, or a functional part, derivative and/or analogue thereof.

11. Use according to any of claims 1-10, whereby said substance is capable of enhancing endogenous expression of DnaJB8 protein, or a functional equivalent thereof.

12. Use according to claim 11, whereby said substance comprises testosterone or a functional part, derivative and/or analogue thereof.

13. Method for determining whether a candidate substance is capable of counteracting protein aggregation, comprising determining whether said candidate compound is capable of enhancing the amount and/or activity of DnaJB8, or a functional part, derivative and/or analogue thereof.

14. Method for determining whether a candidate substance is capable of counteracting protein aggregation, comprising determining whether said candidate substance is capable of enhancing expression and/or activity of DnaJB8, or a functional part, derivative and/or analogue thereof.

15. Method for determining whether a substance is capable of enhancing the level of expression of DnaJB8, or a functional part, derivative and/or analogue thereof, the method comprising:
a. providing a plurality of substances;
b. providing at least one nucleic acid expression system comprising at least one nucleic acid molecule comprising a promoter region of DnaJB8 that is operationally linked to a reporter gene;
c. contacting said at least one nucleic acid expression system with said plurality of substances;
d. measuring expression of said reporter gene; and
e. determining whether expression of said reporter gene is enhanced in the presence of a substance, as compared to the expression of said reporter gene in the absence of said substance, whereby an enhanced expression of said reporter gene in the presence of said substance demonstrates that said substance is capable of enhancing the level of expression of DnaJB8, or a functional part, derivative and/or analogue thereof.

16. Method according to any one of claims 13-15, further comprising selecting a candidate substance which is capable of enhancing expression and/or activity of DnaJB8, or a functional part, derivative and/or analogue thereof.

17. Method for determining whether a candidate substance is capable of enhancing a type II Hsp40 protein's capability of counteracting protein aggregation without interaction with or upregulation of a member of the HSP-70 family, the method comprising determining whether the anti protein aggregation activity of said type II Hsp40 protein, or of a functional part, derivative and/or analogue thereof, is enhanced in the presence of said candidate compound, as compared to the anti protein aggregation activity of said type II Hsp40 protein or functional part, derivative and/or analogue in the absence of said candidate compound, in a manner independent of interaction with or upregulation of members of the HSP-70 protein family.

18. Method for determining whether a candidate substance is, without requiring interaction with or upregulation of members of the HSP-70 protein family, capable of enhancing the anti- polyglutamine-mediated protein aggregation activity of a type II Hsp40 protein, or of a functional part, derivative and/or analogue thereof, the method comprising:
a. providing a plurality of substances;
b. providing a multitude of receptacles, each comprising a mixture comprising a proteinaceous molecule comprising a stretch of more than 20 glutamines, and a type II Hsp40 protein;
c. adding said plurality of substances to said multitude of receptacles;
d. determining whether a substance is capable of decreasing the aggregation of said proteinaceous molecule in a manner independent of interaction with or upregulation of members of the HSP-70 protein family.

19. Method according to claim 17 or 18, wherein said type II Hsp40 protein comprises DnaJB8 protein and/or DnaJB6 protein.

20. Method according to any one of claims 17-19, further comprising selecting a candidate substance which is capable of enhancing the capability of a type II Hsp40 protein, or the capability of a functional part, derivative and/or analogue of a type II Hsp40 protein, of counteracting protein aggregation without interaction with or upregulation of a member of the HSP-70 family.

21. Method for treating an individual suffering from, or at risk of suffering from, a disorder associated with protein aggregation, the method comprising administering to said individual a pharmaceutically effective amount of a substance that is capable of enhancing the amount and/or activity of DnaJB8, or a functional equivalent thereof, in said individual.
